# EUROPEAN PATENT SPECIFICATION

(11) **EP 3 674 381 B1**
(45) Date of publication and mention of the grant of the patent: **10.05.2023**
(21) Application number: 18848811.8
(22) Date of filing: 24.08.2018
(51) Int. Cl.: C09K 11/06, C07D 213/18, C07D 213/30, C07D 333/22, C07F 5/06, C07F 5/02, C07F 7/18, C07F 15/02, C07F 5/00, C07F 1/08, C07D 335/16, C07C 255/37, C07D 311/82, C07D 219/06, C07D 401/04, C07D 311/30, C07C 255/36, C07C 255/58, C07D 211/86, C07D 311/18

(54) **ORGANIC OPTICAL MATERIAL**
ORGANISCHES OPTISCHES MATERIAL
MATÉRIAU OPTIQUE ORGANIQUE

(30) Priority: 25.08.2017 JP 2017161912; 02.03.2018 JP 2018037356
(43) Date of publication of application: 01.07.2020
(73) Proprietor: Japan Science and Technology Agency, Kawaguchi-shi, Saitama 332-0012 (JP)
(72) Inventor: KUNINOBU, Yoichiro, Kasuga-shi Fukuoka 816-8580 (JP); KANAI, Motomu, Tokyo 113-0033 (JP); YAMAKAWA, Takeshi, Shenzhen City, Guangdong 518000 (CN); YOSHIGOE, Yusuke, Tsukuba-shi, Ibaraki, 3058565 (JP); WANG, Zijia, Gusngzhou City, Guangdong 510006 (CN); NAGASHIMA, Hideo, Kasuga-shi Fukuoka 816-8580 (JP); TAHARA, Atsushi, Kasuga-shi Fukuoka 816-8580 (JP)
(74) Representative: Blodig, Wolfgang
(86) International application number: PCT/JP2018/031419
(87) International publication number: WO 2019/039597

(56) References cited:
- WO-A1-98/56765
- WO-A1-2014/132704
- WO-A1-2017/016653
- CN-A- 105 542 753
- CN-A- 105 742 514
- CN-A- 106 147 752
- JP-A- 2000 504 298
- JP-A- 2005 171 221
- JP-A- 2015 187 100
- SAKAI KOJI ET AL: "Crystal structure and nonlinear optical properties of stilbazolium derivatives having shortened cut-off wavelength", PROCEEDINGS OF SPIE, vol. 1337, 1 December 1990 (1990-12-01), pages 307-313, XP055821874, US ISSN: 0277-786X, DOI: 10.1117/12.22957 ISBN: 978-1-5106-4374-1
- PUTTREDDY RAKESH ET AL: "Very strong - N-X + ... - O-N + halogen bonds", CHEMICAL COMMUNICATIONS, vol. 52, no. 11, 1 January 2016 (2016-01-01), pages 2338-2341, XP055821897, ISSN: 1359-7345, DOI: 10.1039/C5CC09487A
- MAKHOTKINA OLENA ET AL: "Cocrystal or Salt: Solid State-Controlled Iodine Shift in Crystalline Halogen-Bonded Systems", CRYSTAL GROWTH & DESIGN, vol. 15, no. 7, 1 July 2015 (2015-07-01), pages 3464-3473, XP055821899, US ISSN: 1528-7483, DOI: 10.1021/acs.cgd.5b00535
- VOGLER ARND ED - PAN MEI: "Luminescence of some simple tetracoordinate organoboron complexes in solution. Fluorescence from ligand to ligand CT states", INORGANIC CHEMISTRY COMMUNICATIONS, ELSEVIER , AMSTERDAM, NL, vol. 72, 26 August 2016 (2016-08-26), pages 83-85, XP029723606, ISSN: 1387-7003, DOI: 10.1016/J.INOCHE.2016.08.012
- LESLEY M J G ET AL: "LEWIS ACIDIC BORANE ADDUCTS OF PYRIDINES AND STILBAZOLES FOR NONLINEAR OPTICS", CHEMISTRY OF MATERIALS, AMERICAN CHEMICAL SOCIETY, US, vol. 10, no. 5, 1 January 1998 (1998-01-01), pages 1355-1365, XP001002291, ISSN: 0897-4756, DOI: 10.1021/CM9707519
- NISHIDA, T. et al.: "Synthesis of Pyridine N-Oxide-BF2CF3 Complexes and Their Fluorescence Properties", CHEMISTRY AN ASIAN JOURNAL, vol. 9, no. 4, February 2014 (2014-02), pages 1026-1030, XP055577505,

## Description

### Field of the Invention

The present invention relates to an organic optical material which intensely emit light not only in a solution state but in a solid state.

### Background of the Invention

Light emitting organic compounds are used in a wide range of fields such as biological labeling materials, organic electronics materials, chemical sensors, and organic lasers. Novel light emitting materials are under active research and development. In particular, fluorescent organic compounds which emit light by irradiation with X-ray, ultraviolet ray, or visible light may be utilized in organic fluorescent coatings, etc., in addition to the purposes described above.

However, most of molecules generally used as light emitting organic materials are robust and highly planar molecules. Therefore, such molecules intensely emit light in a solution because of less contact or interference between the molecules. By contrast, the molecules in a solid state such as a crystalline state often drastically reduce light emission efficiency because the energy of a released electromagnetic wave is attenuated by the influence of its neighboring planar molecules. Since the light emitting organic materials may be used in a solid state in terms of application, an important challenge thereto is to develop materials which intensely emit light not only in a solution but in a solid state.

Many donor-acceptor type compounds have been reported and are known to exhibit diverse characteristics.

Patent Literature 1 discloses a fluorescence emitting compound comprising a nitrogen-containing aromatic ring having high light emission efficiency not only in a solution state but in a solid state.

Non Patent Literature 1 states that the fluorescence wavelength of a so-called donor-acceptor type compound in which the donor and the acceptor are conjugated with each other can be controlled by causing hydrochloric acid, trifluoroacetic acid, or boron tribromide as an activator to react with the compound.

Non Patent Literature 2 describes various analyses conducted by allowing a boron-containing compound to act on a compound consisting of a dimethylamino group and a pyridine ring, which results in a finding that a dipole moment is increased through the reaction of the nitrogen atom of the pyridine ring with the boron atom. However, the literature does not disclose influence on a fluorescence wavelength.

### Citation List

### Patent Literature

Patent Literature 1: International Publication No. WO 2014/132704

### Non Patent Literature

Non Patent Literature 1: Chem. Sci. 2013, 4, 3288
Non Patent Literature 2: Chem. Mater. 1998, 10, 1355
JP 2015 187100 A discloses an organic microcrystal oriented dispersion.
WO 2017/016653 A1 discloses compounds exhibiting photophysical properties upon formation of Lewis acid-base adducts using non-chelating boranes.
Sakai Koji et al., Proceedings of Spie, Vol. 1337, pages 307-313, disclose crystal structure and nonlinear optical properties of stilbazolium derivatives.
Puttredy Rakesh et al., Chemical Communications, 52, 2338-2341 disclose very strong -N-X+···-O-N+ halogen bonds. Makhotkina Olena et al., Crystal Growth & Design, 15, 3464-3473 disclose solid state-controlled iodine shift in crystalline halogen-bonded systems.
Vogler Arnd, Inorganic Chemistry Communications, 72, 83-85 discloses luminescence of some simple tetracoordinate organoboron complexes in solution
JP 2005 171221 A discloses an organic microcrystal oriented dispersion.
CN 105 542 753 A discloses organic nanocrystals.
CN 106 147 752 A discloses a fluorescent probe and a manufacturing method thereof.
CN 105 742 514 a method to improve the alcohol/water solubility and the interface modification characteristics of a traditional evaporation-type electron transport material.
WO 98/56765 A1 discloses chemoluminescent acridinium derivatives.

### Summary of the Invention

### Problem to be Solved by the Invention

An object of the present invention is to provide a novel donor-acceptor type organic optical material which emits light even in a solid state.

### Means for Solving the Problem

In order to attain the object, the present inventors reacted a compound of the pi-conjugated system comprising an electron donating site acting as a donor and an electron accepting site acting as an acceptor, with a compound having a proton donating property or an electron pair accepting property, such as a Bronsted acid, a Lewis acid, or a substance producing a halogen bonding crosslinked structure. As a result, the present inventors found that: a complex is easily produced at the electron accepting site through a non-covalent interaction; and the complex emits light not only in a solution state but in a solid state and is useful as an organic optical material, thereby achieving the completion of the present invention.

Specifically, the present invention relates to the following aspects [1] to [6].
[1] An organic optical material comprising a complex formed from (1) a conjugated molecule having (a) at least one electron donating site, (b) at least one electron accepting site, and (c) at least one conjugated site in the same molecule and (2) a compound having a proton donating property or an electron pair accepting property, the complex having a non-covalent interaction at the electron accepting site, wherein
   the conjugated molecule does not have both one hydrogen atom and one disubstituted amino group at the same time on one of carbon atoms constituting an aliphatic carbon-carbon double bond in its molecule; the complex is solid at ordinary temperature; and the organic optical material exhibits a property of emitting light having a maximum fluorescence wavelength which causes a Stokes shift having a value corresponding to 5% or more of the value of a maximum absorption wavelength, or of 15 nm or more or 1.5 × 10³ cm⁻¹ or more from the maximum absorption wavelength toward the long wavelength side when the conjugated molecule and the compound having a proton donating property or an electron pair accepting property form the complex, wherein the complex is a complex represented by the following formula (1a):

   W:X ... (1a)

   wherein W represents a conjugated molecule represented by the compositional formulas (A)ₗ, (B)ₘ and (C)ₙ; each A is the same or different and represents an electron donating site selected from a hydroxy group, an alkoxy group, an alkenyl group, a silyloxy group, a silyl group, an amino group, a monosubstituted amino group, a disubstituted amino group, an alkoxycarbonyl group, a monovalent or divalent aromatic hydrocarbon group, a monovalent or divalent fused polycyclic aromatic hydrocarbon group, a monovalent or divalent saturated or unsaturated heterocyclic group, or a monovalent or divalent saturated or unsaturated fused polycyclic heterocyclic group, wherein at least one hydrogen atom of each of these groups is optionally replaced with a substituent, and when a plurality of the same or different groups are present as to each of these groups, the groups are optionally linked through a divalent atom or a divalent substituent ; each B is the same or different and represents a conjugated site, selected from an alkenylene group, an alkynylene group, or a substituent in which at least one of these substituents is bonded to a phenylene group; each C is the same or different and represents an electron accepting site, selected from (1) a halogen atom, (2) a nitro group, a phenylamino group, carbene, N-heterocyclic carbene, silylene, N-heterocyclic silylene, nitrene, N-heterocyclic nitrene, a BF₂ group, a cyano group, a pyridyl group, a pyridine N-oxy group, an acridine ring group, an acridine N-oxy group, a diazo group, a triazole group, a benzotriazole group, an oxazole group, a benzoxazole group, a thiazole group, a benzothiazole group, a diazole group, a benzodiazole group, an amino group, a monosubstituted amino group, a disubstituted amino group, a pyrazine ring group, a phenazine ring group, a pyrrolidone ring group, a pyridone ring group, an aromatic heterocyclic group, or a fused polycyclic aromatic heterocyclic group, or (3) a formyl group, a carbonyl group, a carbodiimide group, a pyrrolidone ring group, a pyridone ring group, a formyl group, a quinone ring group, a quinoline ring group, a naphthoquinone ring group, an anthraquinone ring group, a lactone ring group, or an oxycarbonyl group; : represents a non-covalent interaction; X represents a compound having a proton donating property or an electron pair accepting property, which is selected from p-toluenesulfonic acid, trifluoromethanesulfonic acid, methanesulfonic acid, N,N-dialkylimidazolinium-2-halide, halogenoisoindolyl-1,3-dione, 2-halogenobenz[d]isothiazol-3(2H)-one 1,1-dioxide, 2-halogeno-5-nitroisoindolyl-1,3-dione, or 2-halogeno-3,4-dimethylthiazol-3-inium trifluorosulfonate; l and n each represent a number of 1 to 6; m represents a number of 1 to 6; and C and X are non-covalently bonded.
[2] The organic optical material according to of [1], wherein the complex is a complex represented by the following formula (1b):

   W:X ... (1b)

   wherein W represents a conjugated molecule consisting of (A)ₗ₁-(B)ₘ₁-(C)ₙ₁, (C)ₙ₂-(B)ₘ₂-(A)₁₂-(B)ₘ₃-(A)ₗ₁-(B)ₘ₁-(C)ₙ₁ or (A)ₙ₂-(B)ₘ₂-(C)ₗ₂-(B)ₘ₃-(C)ₗ₁-(B)ₘ₁-(A)ₙ₁; A represents an electron donating site as defined in [1]; B represents a conjugated site as defined in [1]; C represents an electron accepting site as defined in [1]; : represents a non-covalent interaction as defined in [1]; X represents a compound having a proton donating property or an electron pair accepting property as defined in [1]; l1 and n1 each represent 1; m1 represents a number of 0 to 2; l2 and n2 each represent 1; m2 represents a number of 0 to 2; m3 represents 1; and C and X are non-covalently bonded.
[3] Use of an organic optical material according to [1] or [2] as an optical material which is contained in an optical composition selected from the composition group consisting of a fluorescence emitting composition, a fluorescent coating composition, a wavelength conversion member composition, an organic EL member composition, an organic laser member composition, an organic transistor member composition, an organic solar cell member composition, and a chemical sensor member composition.
[4] A mixture comprising two or more organic optical materials according to [1] or [2] mixed with each other, or an organic optical material according to [1] or [2] mixed with an additional organic optical material.
[5] A method for adjusting the color of emitted light, comprising using one or two or more organic optical materials according to [1] or [2], or a mixture of the one or two or more organic optical materials with an additional organic optical material.
[6] A fluorescent agent comprising a complex according to [1].

### Effects of the Invention

The complex of the present invention intensely emits light not only in a solution state but in a solid state. A complex having strong fluorescence intensity can be easily obtained, without a complicated structure, by merely reacting a conjugated compound having an electron donating site and an electron accepting site in the same molecule with a compound such as a Bronsted acid. The complex compound has a longer maximum emission wavelength, a higher fluorescence quantum yield, and a prolonged fluorescence lifetime as compared with the conjugated molecule before formation of the complex. Thus, the complex of the present invention is useful as an organic optical material which is contained in an optical composition selected from the composition group consisting of a fluorescence emitting composition, a fluorescent coating composition, a wavelength conversion member composition, an organic EL member composition, an organic transistor member composition, an organic solar cell member composition, and a composition for a chemical sensor member and an organic laser member.

In the present invention, the organic optical material refers to a general material which exploits the conversion of energy levels of electrons constituting the material in response to light. Such a material exerts its functions in response to light and, for example, produces color or emits fluorescence. Specifically, the organic optical material can therefore be used as organic EL, organic solid laser, an organic non-linear optical material, ink, and the like, and in addition, can be used in an organic solar cell, an organic solid sensor, and the like. Furthermore, the organic optical material can also be used in an opto-electronics material such as a photoresponsive electron transport layer, an organic waveguide, a diode, or a transistor.

### Brief Description of the Drawings

[Figure 1] Figure 1 shows an emission spectrum of a compound obtained in Example 1.
[Figure 2] Figure 2 shows an emission spectrum of a compound obtained in Example 8.
[Figure 3] Figure 3 shows an emission spectrum of a compound obtained in Example 13.
[Figure 4] Figure 4 shows an emission spectrum of a compound obtained in Example 2.
[Figure 5] Figure 5 shows an emission spectrum of a compound obtained in Example 9.
[Figure 6] Figure 6 shows an emission spectrum of a compound obtained in Example 11.
[Figure 7] Figure 7 shows an emission spectrum of a compound obtained in Example 14.
[Figure 8] Figure 8 shows an emission spectrum of a compound obtained in Example 3.
[Figure 9] Figure 9 shows an emission spectrum of a compound obtained in Example 10.
[Figure 10] Figure 10 shows an emission spectrum of a compound obtained in Example 12.
[Figure 11] Figure 11 shows an emission spectrum of a compound obtained in Example 15.
[Figure 12] Figure 12 shows an emission spectrum of a compound obtained in Example 7.
[Figure 13] Figure 13 shows an emission spectrum of a compound obtained in Example 16.
[Figure 14] Figure 14 shows an emission spectrum of a compound obtained in Example 17.
[Figure 15] Figure 15 shows an emission spectrum of a compound obtained in Example 18.
[Figure 16] Figure 16 shows an emission spectrum of a compound obtained in Example 19.
[Figure 17] Figure 17 shows an emission spectrum of a compound obtained in Example 20.
[Figure 18] Figure 18 shows an emission spectrum of a compound obtained in Example 21.
[Figure 19] Figure 19 shows an emission spectrum of a compound obtained in Example 22.
[Figure 20] Figure 20 shows emission spectra in a solution of the compounds obtained in Examples 1 and 8.
[Figure 21] Figure 21 shows emission spectra in a solution of the compounds obtained in Examples 1 and 13.
[Figure 22] Figure 22 shows emission spectra in a solution of the compounds obtained in Examples 2 and 9.
[Figure 23] Figure 23 shows emission spectra in a solution of the compounds obtained in Examples 2 and 11.
[Figure 24] Figure 24 shows emission spectra in a solution of the compounds obtained in Examples 2 and 14.
[Figure 25] Figure 25 shows emission spectra in a solution of the compounds obtained in Examples 10 and 15.
[Figure 26] Figure 26 shows emission spectra in a solution of the compounds obtained in Examples 3 and 12.
[Figure 27] Figure 27 shows emission spectra in a solution of the compounds obtained in Examples 3 and 15.
[Figure 28] Figure 28 shows emission spectra in a solution of the compounds obtained in Examples 7 and 16.

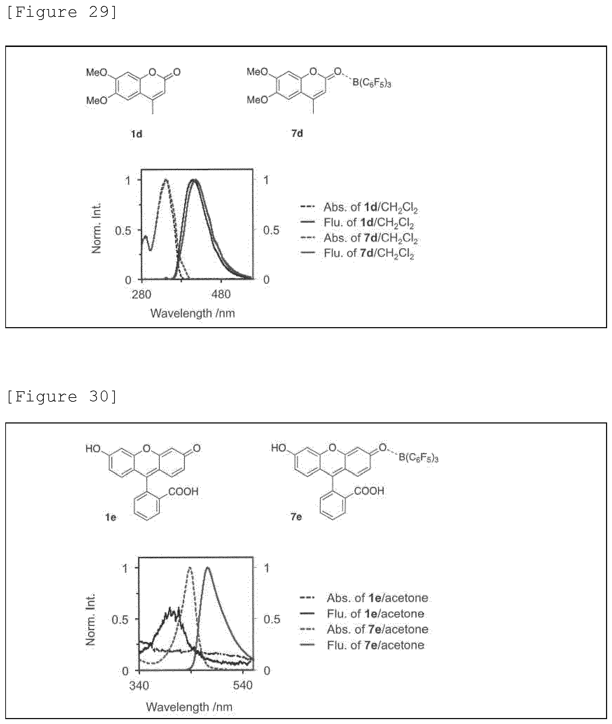

[Figure 29] Figure 29 shows an emission spectrum in a solvent of the compound obtained in Example 17.
[Figure 30] Figure 30 shows an emission spectrum in a solvent of the compound obtained in Example 18.

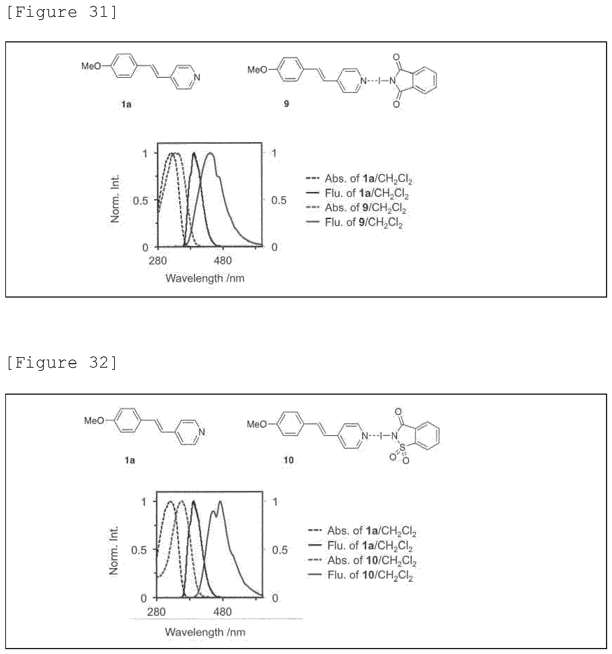

[Figure 31] Figure 31 shows an emission spectrum in a solvent of the compound obtained in Example 19.
[Figure 32] Figure 32 shows an emission spectrum in a solvent of the compound obtained in Example 20.

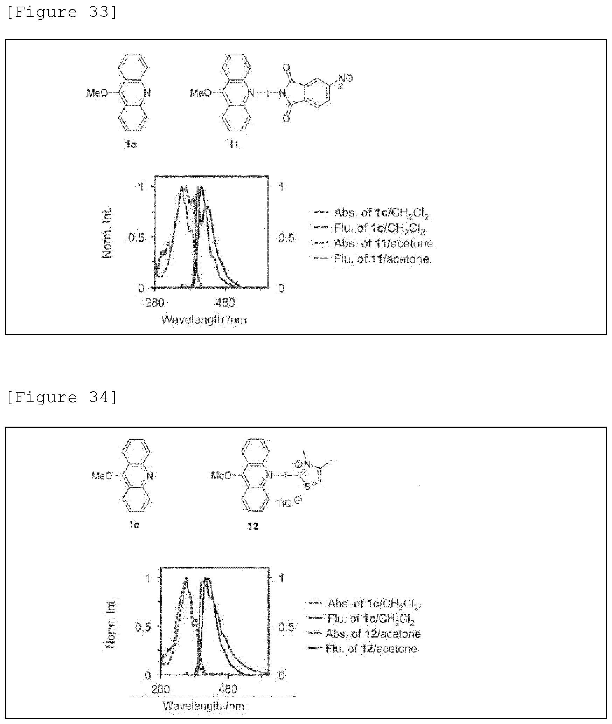

[Figure 33] Figure 33 shows an emission spectrum in a solvent of the compound obtained in Example 21.
[Figure 34] Figure 34 shows an emission spectrum in a solvent of the compound obtained in Example 22.
[Figure 35] Figure 35A shows 2,3-bis(4-((triisopropylsilyl)oxy)phenyl)fumaronitrile and Figure 35B shows 2,3-bis(4-((triisopropylsilyl)oxy)phenyl)fumaronitrile and tris(pentafluorophenyl)borane. In the drawings, crystal structures determined by X-ray structural analysis before complex formation (figure 35A) and after complex formation (figure 35B) are presented in an associated state of two molecules in terms of models when a double bond is laterally viewed. For simplification, however, hydrogen atoms are omitted.

### Detailed Description of the Invention

The complex of the present invention is a pi-conjugated compound complex comprising a complex formed from (1) a conjugated molecule having (a) at least one electron donating site, (b) at least one electron accepting site, and (c) at least one conjugated site in the same molecule and (2) a compound having a proton donating property or an electron pair accepting property, the complex having a non-covalent interaction at the electron accepting site.

However, for the organic optical material of the present invention it is excluded that the conjugated molecule is a conjugate molecule having both of one hydrogen atom and one disubstituted amino group at the same time on one of carbon atoms constituting an aliphatic carbon-carbon double bond in its molecule.

The complex of the present invention is solid at ordinary temperature. In this context, the ordinary temperature refers to a temperature in the range of from 5 to 35°C.

In the complex, one or more long-wavelength shifts, so-called Stokes shifts, from a maximum absorption wavelength toward the long wavelength side are observed even in a solid state, as compared with the conjugated molecule before formation of the complex. When the Stokes shift is represented by fluorescence wavelength (nm) - maximum absorption wavelength (≈ excitation wavelength) (nm), the size thereof is 15 nm or more, preferably 30 nm or more, more preferably 60 nm or more. When the size of the Stokes shift (nm) is represented by a size with respect to a fluorescence wavelength (nm), the size is 5% or more, preferably 15% or more, more preferably 20% or more, of the value of the maximum absorption wavelength. The Stokes shift originally refers to a difference in energy and is a function of a wave number. As the Stokes shift may typically have to be indicated in wave number, the size is represented by (1 / fluorescence wavelength) (cm⁻¹) - (1 / maximum absorption wavelength) (cm⁻¹). This Stokes shift is 1.5 × 10³ cm⁻¹ or more, preferably 3.0 × 10³ cm⁻¹ or more, more preferably 4.5 × 10³ cm⁻¹ or more. If fluorescence is difficult to observe in a solid state, a measurement using a solution can be performed instead.

In this context, the Stokes shift occurs by light having at least one excitation wavelength in the range of from 280 nm to 400 nm.

Thus, the complex of the present invention is useful as various organic light emitting materials which exploit a chemical phenomenon in which energy levels of electrons constituting the material are converted in response to light.

The fluorescence quantum yield of the complex in a solid state is 0.01 or more, preferably 0.1% or more, more preferably 0.15% or more, by at least one excitation wavelength in the range of from 280 nm to 400 nm.

The conjugated molecule (1) having (a) at least one electron donating site, (b) at least one electron accepting site, and (c) at least one conjugated site in the same molecule is a molecule with a conjugated pi bond as a rule and is a compound having at least one electron donating site and at least one electron accepting site. The electron donating site and the electron accepting site of the present invention form one pi-conjugated system, and these two sites may be bridged through a pi-conjugated structure. The electron donating site and the electron accepting site may be bonded directly.

The conjugated molecule constituting a portion of the complex of the present invention can be produced in accordance with an approach known in the art or Examples mentioned later, etc.

The "electron accepting site" of the present invention refers to a site at which a non-covalently bonded complex is formed by the direct action of a compound having a proton donating property or an electron pair accepting property on the site. In the present invention, the electron to be donated in this manner is an electron involved in a pi-resonance structure and may bias the electron density of the whole molecule in a state where a non-covalently bonded complex has been formed. When the electron accepting site is locally present in a portion of the chemical structure, a structure where an electron is donated from its surrounding structure may be adopted.

Thus, for example, an aromatic hydrocarbon or an aromatic heterocycle serves as a conjugated system and is also capable of becoming either the electron donating site or the electron accepting site. Therefore, the organic optical material of the present invention is a compound represented by the following formula (1a):

W:X ... (1a)

wherein W represents a conjugated molecule consisting of (A)ₗ, (B)ₘ and (C)ₙ; each A is the same or different and represents an electron donating site; each B is the same or different and represents a conjugated site; each C is the same or different and represents an electron accepting site; : represents a non-covalent interaction; X represents a compound having a proton donating property or an electron pair accepting property; l and n each represent a number of 1 to 6; m represents a number of 1 to 6; and C and X are non-covalently bonded.

W in the formula (1a) is a conjugated molecule consisting of (A)ₗ, (B)ₘ and (C)ₙ. This W may be described by a so-called chemical compositional formula of l:m:n as the composition of A, B and C, or may be represented by a chemical structure of (A)l-(B)m-(C)n. More specifically, W can be illustrated by

A-B-C

or

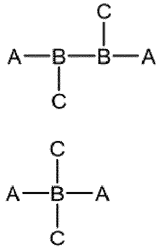

Each electron donating site (A) is the same or different and is a hydroxy group, an alkoxy group, an alkenyl group, a silyloxy group, a silyl group, an amino group, a monosubstituted amino group, a disubstituted amino group, an alkoxycarbonyl group, a monovalent or divalent aromatic hydrocarbon group, a monovalent or divalent fused polycyclic aromatic hydrocarbon group, a monovalent or divalent saturated or unsaturated heterocyclic group, or a monovalent or divalent saturated or unsaturated fused polycyclic heterocyclic group, wherein at least one hydrogen atom of each of these groups is optionally replaced with a substituent, and when a plurality of the same or different groups are present as to each of these groups, the groups are optionally linked through a divalent atom or a divalent substituent.

Examples of the alkoxy group include linear or branched alkoxy groups having 1 to 8 carbon atoms. Specific examples thereof include a methoxy group, an ethoxy group, a n-propyloxy group, an isopropyloxy group, a butyloxy group, a pentyloxy group, and a hexyloxy group.

Examples of the alkyl group include linear or branched alkyl groups having 1 to 8 carbon atoms. Specific examples thereof include a methyl group, an ethyl group, a n-propyl group, an isopropyl group, a n-butyl group, a tert-butyl group, a n-pentyl group, and a n-hexyl group.

Examples of the alkenyl group include linear or branched alkenyl groups having 2 to 8 carbon atoms. Specific examples thereof include a vinyl group and a propenyl group.

Examples of the silyloxy group include trialkylsilyloxy groups, for example, a trimethylsilyloxy group and a triisopropylsilyloxy group.

Examples of the monosubstituted amino group include alkylamino groups and arylamino groups. Specific examples thereof include a methylamino group, an ethylamino group, a propylamino group, and a phenylamino group. Examples of the disubstituted amino group include dialkylamino groups and diarylamino groups. Specific examples thereof include a dimethylamino group, a dipropylamino group, and a diphenylamino group.

Examples of the monovalent or divalent aromatic hydrocarbon group and the monovalent or divalent fused polycyclic aromatic hydrocarbon group include a phenyl group, a phenylene group, an indene ring group, a naphthyl group, a naphthalene ring group, a phenylphenylene group (a biphenyl group), an anthracene ring group, a phenanthrene ring group, a naphthacene ring group, a triphenylene ring group, a pyrene ring group, a chrysene ring group, and a perylene ring group.

Examples of the monovalent or divalent saturated or unsaturated heterocyclic group and the monovalent or divalent saturated or unsaturated fused polycyclic heterocyclic group include a pyrrole ring group, a furan ring group, a thiophene ring group, an oxazole ring group, a thiazole ring group, a diazole ring group, a triazole ring group, a pyrimidine ring group, a pyrazine ring group, a piperidine ring group, a piperazine ring group, a benzoxazole ring group, a benzothiazole ring group, an indole ring group, a quinoline ring group, an isoquinoline ring group, a benzothiadiazole ring group, and a porphyrin ring group.

In the electron donating group, at least one hydrogen atom may be replaced with a substituent (e.g., an alkyl group, an alkoxy group, and a halogen atom). The substituent is preferably an electron donating group. When a plurality of groups are present as to each of these groups, the groups may be linked through a divalent atom (e.g., an oxygen atom and a sulfur atom) or a divalent substituent (e.g., methylene and vinylene).

Each A is the same or different and preferably represents a hydroxy group, an alkoxy group, an alkenyl group, a silyloxy group, a silyl group, an amino group, an alkylamino group, a dialkylamino group, an arylamino group, a diarylamino group, a pyrrole group, a phenyl group, a phenylene group, a phenylphenylene group, an anthracene ring group, a phenanthrene ring group, a naphthacene ring group, a triphenylene ring group, a pyrene ring group, a chrysene ring group, a perylene ring group, an oxazole ring group, a thiazole ring group, a porphyrin ring group, a piperazine ring group, or an alkoxycarbonyl group, wherein at least one hydrogen atom of each of these groups is optionally replaced with a substituent, and when a plurality of the same or different groups are present as to each of these groups, the groups are optionally linked through a divalent substituent.

Each A is the same or different and more preferably represents a hydroxy group, an optionally alkoxy group-substituted alkoxy group, an optionally alkyl group-substituted silyloxy group, an optionally hydroxy group-substituted phenyl group, a phenylphenylene group, or a substituent having an electron donating property in which at least one hydrogen atom of an aromatic hydrocarbon group is optionally replaced with a substituent.

In the formula (1a), each conjugated site (B) is the same or different and preferably represents a conjugated site having a chemical structure where multiple bonds are linked through a carbon-carbon single bond.

Each B is the same or different and represents an alkenylene group, an alkynylene group, or a substituent in which at least one of these substituents is bonded to a phenylene group.

Each B is the same or different and more preferably represents an alkenylene group, or an optionally diphenylene-substituted vinylene group. More specific examples thereof include vinylene, 1,3-butadienylene, acetylene, and 1,3-diacetylene.

Each electron accepting site (C) is the same or different and is
(1) a halogen atom,
(2) a nitro group, a phenylamino group, carbene, N-heterocyclic carbene, silylene, N-heterocyclic silylene, nitrene [R-N(:):], N-heterocyclic nitrene, a BF₂ group, a cyano group, a pyridyl group, a pyridine N-oxy group, an acridine ring group, an acridine N-oxy group, a diazo group, a triazole group, a benzotriazole group, an oxazole group, a benzoxazole group, a thiazole group, a benzothiazole group, a diazole group, a benzodiazole group, an amino group, a monosubstituted amino group, a disubstituted amino group, a pyrazine ring group, a phenazine ring group, a pyrrolidone ring group, a pyridone ring group, an aromatic heterocyclic group, or a fused polycyclic aromatic heterocyclic group, or
(3) a formyl group, a carbonyl group, a carbodiimide group, a pyrrolidone ring group, a pyridone ring group, a formyl group, a quinone ring group, a quinoline ring group, a naphthoquinone ring group, an anthraquinone ring group, a lactone ring group, or an oxycarbonyl group.

Each C is the same or different and preferably represents a cyano group, a pyridyl group, an acridine ring group, or a lactone ring group.

For the form of W, a complex represented by the following formula (1b) is preferred:

W:X ... (1b)

wherein W represents a conjugated molecule consisting of (A)ₗ₁-(B)ₘ₁-(C)ₙ₁, (C)ₙ₂-(B)ₘ₂-(A)ₗ₂-(B)ₘ₃-(A)ₗ₁-(B)ₘ₁-(C)ₙ₁ or (A)ₙ₂-(B)ₘ₂-(C)ₗ₂-(B)ₘ₃-(C)ₗ₁-(B)ₘ₁-(A)ₙ₁; A represents an electron donating site as defined above; B represents a conjugated site as defined above; C represents an electron accepting site as defined above; : represents a non-covalent interaction; X represents a compound having a proton donating property or an electron pair accepting property as defined herein; l1 and n1 each represent 1; m1 represents a number of 0 to 2; l2 and n2 each represent 1; m2 represents a number of 0 to 2; m3 represents 1; and C and X are non-covalently bonded.

Among the conjugated molecules represented by W, examples of the A-B-C type compound can include the following compounds.

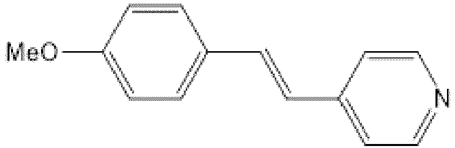

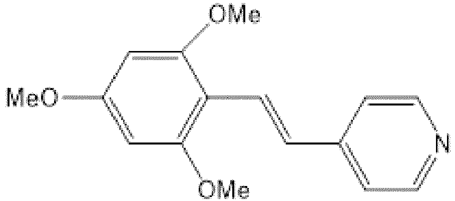

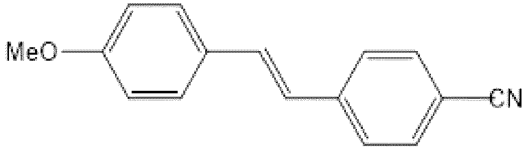

The site C causes a non-covalent interaction with a Lewis acid or the like so that a resonance structure where an electron from the electron donating site A passes through the bridge moiety B is formed to produce a new pi-conjugated system.

Examples of the C-B-A-B-C type compound include the following compound:

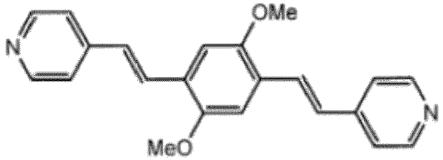

The compound described above has a structure where the pyridine ring corresponds to C; the alkenylene group corresponds to B; the site where the anisole type methoxy group is present at the para position corresponds to A; and two B-C moieties are presumably bridged through A.

Examples of 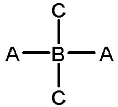 include the following compound:

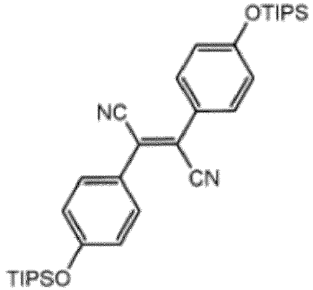

In the compound described above, the nitrile group corresponds to C, the alkenylene group corresponds to B, and the siloxybenzene corresponds to A. The moiety B is a site common in two moieties A and two moieties C.

X is a compound having a proton donating property or an electron pair accepting property and is a compound which causes a non-covalent interaction with the electron accepting site. Specifically, a Bronsted acid, a Lewis acid, and a halogen bonding donor are known. In the present invention, X refers to an electron acceptor in the broad sense. In the present specification, the halogen bonding donor is also referred to as a halogen donor.

The electron acceptor has an unoccupied orbital capable of accepting an electron pair and interacts with the pi-conjugated molecule represented by W so that the electron acceptor accepts an electron pair from the compound to form a complex. Examples of the electron acceptor include Lewis acids as well as cation species. The cation species is typified by a proton or a halogen cation. Usually, the proton is provided by a Bronsted acid, and the halogen cation is provided by a halogen bonding donor.

The halogen cation has an unoccupied orbital for acceptance of an electron pair and can accept an electron pair through interaction with the pi-conjugated molecule represented by W to form a complex. According to the detailed studies, the mechanism can be classified into three types as illustrated below, except for the case where the halogen cation is provided in any manner.
(1) A compound I-Cl in which halogen atoms are bonded to each other is polarized into I(δ+)-Cl(δ-) due to large difference in electronegativity to create a state where an unoccupied orbital for acceptance of an electron pair is present on the iodine. Therefore, the compound forms a complex with the pi-conjugated molecule represented by W.
(2) In I-C6F5, an electron density on the benzene ring is decreased due to large electronegativity of F. A non-covalent electron pair present on the iodine works to compensate for the electron density on the benzene ring. The compound is stabilized by the resonance structure of the strongly positively polarized iodine. Since an unoccupied orbital for acceptance of an electron pair is formed on the iodine, the compound forms a complex with the pi-conjugated molecule represented by W.
(3) Iodoimidazolinium salt is stable because a trifluoromethane sulfonate anion and an imidazole cation form a salt. A non-covalent electron pair present on the iodine works to compensate for the electron density on the imidazole ring. The compound is stabilized by the resonance structure of the strongly positively polarized iodine. Since an unoccupied orbital for acceptance of an electron pair is formed on the iodine, the compound forms a complex with an unpaired electron of the pi-conjugated molecule represented by W.

The compound X having a proton donating property or an electron pair accepting property may form a complex with the pi-conjugated molecule W, thereby exhibiting an effect as an organic optical material. In this case, an effect as described below may be produced. For example, (1) the compound X of the present invention in crystals of its complex with the pi-conjugated molecule W prevents stacking of substituents of W accompanied by quenching due to steric hindrance, etc., or formation of other structures inappropriate for fluorescence observation. (2) The compound X of the present invention in its complex with the pi-conjugated molecule W decreases the difference between HOMO and LUMO energy levels in W after complex formation as compared with the difference between HOMO and LUMO energy levels in W before complex formation.

The present inventors do not think that the mechanisms (1) and/or (2) exhibit specific fluorescence characteristics in all events, but believe that the pi-conjugated molecule W of the present invention can decrease the HOMO-LUMO energy gap through formation of a complex with the compound X having a proton donating property or an electron pair accepting property, and thereby shift a fluorescence wavelength to a longer wavelength than that before complex formation. Specifically, the pi-conjugated molecule W of the present invention can control a fluorescence wavelength through the compound X having a proton donating property or an electron pair accepting property.

Examples of the Bronsted acid include methanesulfonic acid and p-toluenesulfonic acid.

The halogen bonding donor can accept a non-covalent electron pair of a Lewis base functional group to an unoccupied orbital resulting from the structure of the halogen bonding donor itself. Specific examples thereof include N,N-dialkylimidazolinium-2-halide, 2-halogenoisoindolyl-1,3-dione, 2-halogenobenz[d]isothiazol-3(2H)-one 1,1-dioxide, 2-halogeno-5-nitroisoindolyl-1,3-dione, and 2-halogeno-3,4-dimethylthiazol-3-inium trifluorosulfonate.

In the complex of the present invention, the electron accepting site in the conjugated molecule, and the compound having a proton donating property or an electron pair accepting property are bonded through a non-covalent bond. Examples of such a bond include the coordinate bond between a cyano group and a Lewis acid, and the ionic bond or the coordinate bond between a nitrogen atom in an aromatic group (e.g., a nitrogen atom in a pyridine ring) and a Bronsted acid, a Lewis acid, or a halogen bonding donor.

The complex of the present invention excludes p-CH₃O-C₆H₅-CH=CH-C₅H₄NBF₃, p-CH₃O-C₆H₅-CH=CH-C₅H₄NB(C₆F₅)₃, p-(CH₃)₂N-C₆H₅-CH=CH-C₅H₄NBF₃ and p- (CH₃)₂N-C₆H₅-CH=CH-C₅H₄NB(C₆F₅)₃.

Specific examples of the pi-conjugated molecule constituting the complex of the present invention will be shown below.

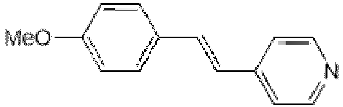

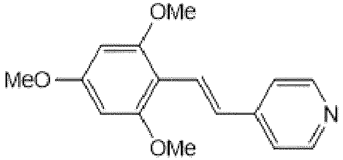

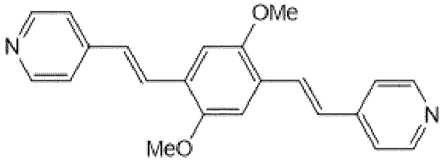

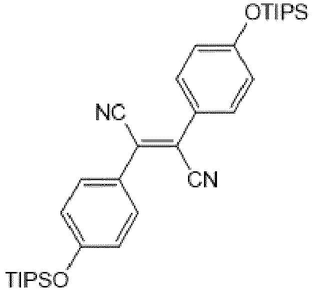

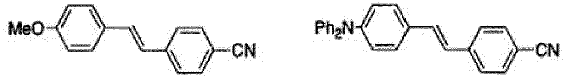

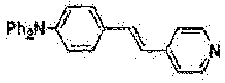

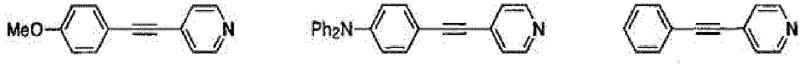

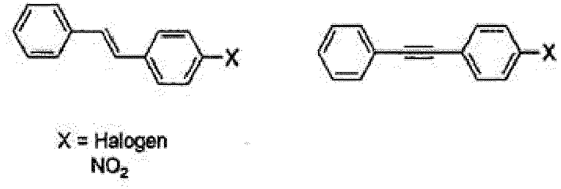

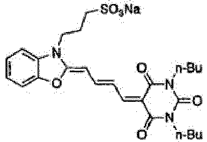

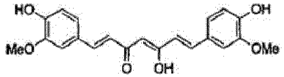

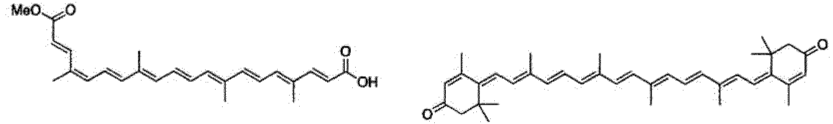

In the complex of the present invention, a preferred combination of A, B, C and X is as follows:

W:X ... (1b)

wherein
W represents a conjugated molecule consisting of (A)ₗ₁-(B)ₘ₁-(C)ₙ₁, (C)ₙ₂-(B)ₘ₂-(A)ₗ₂-(B)ₘ₃-(A)ₗ₁-(B)ₘ₁-(C)ₙ₁ or (A)ₙ₂-(B)ₘ₂-(C)ₗ₂-(B)ₘ₃-(C)ₗ₁-(B)ₘ₁-(A)ₙ₁;
each A is the same or different and represents a hydroxy group, an alkoxy group, an alkenyl group, a silyloxy group, a silyl group, an amino group, a monosubstituted amino group, a disubstituted amino group, an alkoxycarbonyl group, a monovalent or divalent aromatic hydrocarbon group, a monovalent or divalent fused polycyclic aromatic hydrocarbon group, a monovalent or divalent saturated or unsaturated heterocyclic group, or a monovalent or divalent saturated or unsaturated fused polycyclic heterocyclic group, wherein at least one hydrogen atom of each of these groups is optionally replaced with a substituent, and when a plurality of the same or different groups are present as to each of these groups, the groups are optionally linked through a divalent atom or a divalent substituent;
each B is the same or different and represents a conjugated site having a chemical structure where multiple bonds are linked through a carbon-carbon single bond as defined above;
each C is the same or different and represents
   (1) a halogen atom,
   (2) a nitro group, a phenylamino group, carbene, N-heterocyclic carbene, silylene, N-heterocyclic silylene, nitrene, N-heterocyclic nitrene, a BF₂ group, a cyano group, a pyridyl group, a pyridine N-oxy group, an acridine ring group, an acridine N-oxy group, a diazo group, a triazole group, a benzotriazole group, an oxazole group, a benzoxazole group, a thiazole group, a benzothiazole group, a diazole group, a benzodiazole group, a primary amino group, a secondary amino group, a tertiary amino group, a pyrazine ring group, a phenazine ring group, a pyrrolidone ring group, a pyridone ring group, an aromatic heterocyclic group, or a fused polycyclic aromatic heterocyclic group, or
   (3) a formyl group, a carbonyl group, a carbodiimide group, a pyrrolidone ring group, a pyridone ring group, a formyl group, a quinone ring group, a quinoline ring group, a naphthoquinone ring group, an anthraquinone ring group, a lactone ring group, or an oxycarbonyl group;
X represents a compound as defined above;
l1 and n1 each represent 1; m1 represents a number of 0 to 3; l2 and n2 each represent 1; m2 represents a number of 0 to 3; m3 represents 1; and C and X are non-covalently bonded.

A complex of the formula (1b) wherein
W represents a conjugated molecule wherein
   each A is the same or different and is a hydroxy group, an optionally alkoxy group-substituted alkoxy group, an optionally alkyl group-substituted silyloxy group, an optionally hydroxy group-substituted phenyl group, a phenylphenylene group, or a substituent having an electron donating property in which at least one hydrogen atom of an aromatic hydrocarbon group is optionally replaced with a substituent;
   each B is the same or different and is an alkenylene group, or an optionally diphenylene-substituted vinylene group;
   each C is the same or different and is a cyano group, a pyridyl group, an acridine ring group, or a lactone ring group;
   l is 1, 2 or 3; n is 1 or 2; and m is 1;
X represents p-toluenesulfonic acid, trifluoromethanesulfonic acid, methanesulfonic acid, N,N-dialkylimidazolinium-2-halide, 2-halogenoisoindolyl-1,3-dione, 2-halogenobenz[d]isothiazol-3(2H)-one 1,1-dioxide, 2-halogeno-5-nitroisoindolyl-1,3-dione, or 2-halogeno-3,4-dimethylthiazol-3-inium trifluorosulfonate; and
C and X are non-covalently bonded.

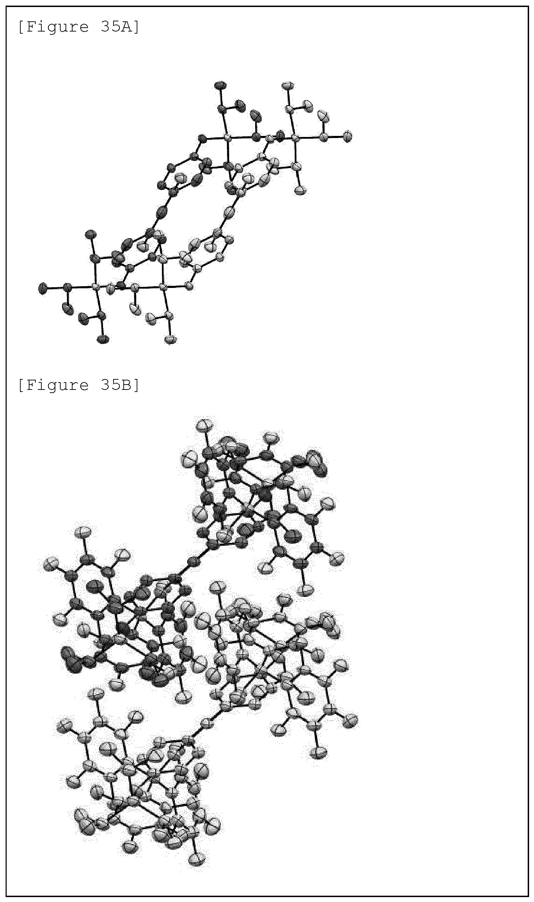

Figure 35A shows 2,3-bis(4-((triisopropylsilyl)oxy)phenyl)fumaronitrile and Figure 35B shows a complex of 2,3-bis(4-((triisopropylsilyl)oxy)phenyl)fumaronitrile and tris(pentafluorophenyl)borane. In the drawings, crystals determined by X-ray structural analysis are presented in an associated state of two molecules. At a stage before complex formation, the stacking of dicyanodiphenylalkene skeletons is suppressed by a bulky OTIPS group. After complex formation, the distance between double bonds is markedly widened by the steric hindrance of the Lewis acid. The Lewis acid is considered to have an effect of decreasing LUMO energy level after complex formation as compared with the LUMO energy level before complex formation, and a shift of the absorption wavelength of visible light to a longer wavelength is observed.

The complex of the present invention emits fluorescence without quenching even in a solid state. Molecules which emit light in a solution emit light in a dilute solution, but no longer emit light when concentrated. This is so-called quenching. The quenching occurs by a radiationless deactivation process such as internal conversion or energy transfer when molecules of the pi-conjugated system come close so as to overlap each other (H-aggregate). Specifically, the stacking of the molecules of the pi-conjugated system through H-association causes a loss of the ability to emit light. On the other hand, it is known that a small mismatch of molecular overlap (J-aggregate) is capable of causing a shift to a longer wavelength and light emission. In addition, it is generally known that since a longer pi-conjugated system causes absorption at a longer wavelength, fluorescence is emitted with a longer wavelength. The complex of the present invention emits light with a longer wavelength by use of a longer pi-conjugated system. The following compounds exhibit particularly marked change in a solid state.

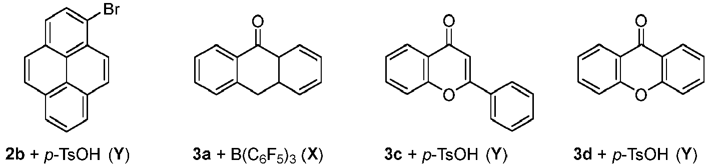

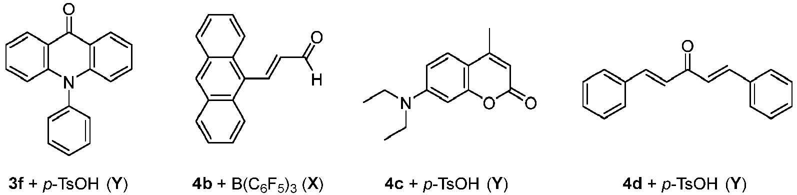

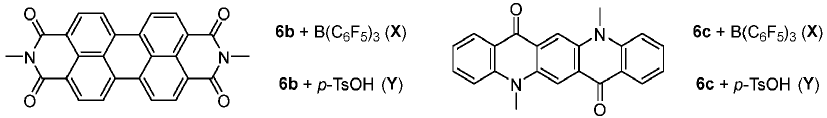

These compounds are considered to directly make no significant change in the resonance structure of the pi-conjugated system by forming a complex with the compound X having a proton donating property or an electron pair accepting property. However, a shift to a longer wavelength probably occurs as the gap between energy levels of the HOMO of a donor and the LUMO of an acceptor is decreased.

The complex of the present invention can be easily produced by reacting the conjugated compound with the Bronsted acid, the Lewis acid, or the halogen bonding donor. The reaction is sufficiently performed by merely stirring the mixture at 0°C to 100°C for 5 minutes to 1 hour in an inert solvent such as methylene chloride.

The obtained complex can be isolated by a usual separation and purification operation such as silica gel column chromatography or recrystallization. The method for producing the complex of the present invention will be specifically described in Examples of the present specification. Those skilled in the art can therefore readily synthesize the complex of the present invention by appropriately selecting starting compounds and reaction conditions with reference to these Examples.

The present invention also provides a method for adjusting the color of emitted light, comprising using one or two or more organic optical materials of the present invention, or a mixture of the one or two or more organic optical materials with an additional organic optical material. One or two or more miscible organic optical materials of the present invention can be selected and mixed by pulverization and kneading methods known in the art to obtain a new complex composition. The color of emitted light can be adjusted by the type, mixing ratio, etc. of the organic optical material of the present invention. Also, the color of emitted light can be adjusted using a composition of the starting materials before complex formation and the organic optical material after complex formation.

The complex of the present invention has a property of emitting intense fluorescence not only in a solution state but in a solid state. In the present specification, the term "fluorescence" means a phenomenon as one mode of light emission in which electrons are excited by absorbing energy upon irradiation with X-ray, ultraviolet ray, and/or visible light, and release extra energy as an electromagnetic wave when returning to the ground state.

The fluorescence should not be interpreted in a limited manner by any means and should be interpreted in the broadest sense. Fluorescence measurement in a solid state can generally be performed using Absolute PL Quantum Yield Spectrometer C-9920-02 (multichannel detector PMA-11, Hamamatsu Photonics K.K.) or the like, though not limited by such a specific apparatus and method.

More specifically, in the complex of the present invention, one or more long-wavelength shifts, so-called Stokes shifts, toward the long wavelength side from a maximum absorption wavelength are observed in a solid state, as compared with the conjugated molecule before formation of the complex, and the size thereof is 5% or more, preferably 15% or more, more preferably 20% or more, of the value of the maximum absorption wavelength. Also, the Stokes shift is 15 nm or more, preferably 30 nm or more, more preferably 60 nm or more. The Stokes shift is further 1.5 × 10³ cm⁻¹ or more, preferably 4.5 × 10³ cm⁻¹ or more, more preferably 3.0 × 10³ cm⁻¹ or more.

In this context, the Stokes shift occurs by light having at least one excitation wavelength in the range of from 280 nm to 400 nm.

Thus, the complex of the present invention is useful as various organic light emitting materials which exploit the conversion of energy levels of electrons constituting the material in response to light.

Specifically, the complex of the present invention can be used as organic EL, organic solid laser, an organic non-linear optical material, ink, and the like, and in addition, can be used in an organic solar cell, an organic solid sensor, and the like. Furthermore, the complex of the present invention can also be used in an opto-electronics material such as a photoresponsive electron transport layer, an organic waveguide, a diode, or a transistor.

### Examples

Hereinafter, the present invention will be described further specifically with reference to Examples. However, the scope of the present invention is not limited by Examples described below.

Examples 2, 3, 4 ,9, 10, 11, 12, 14, 15, 17, 18, 21, 22, 26 to 33, 36, 37, 40 to 43, 45 to 54, and 56 to 83 are Comparative Examples.

### [Example 1] (E)-4-(4-Methoxystyryl)pyridine

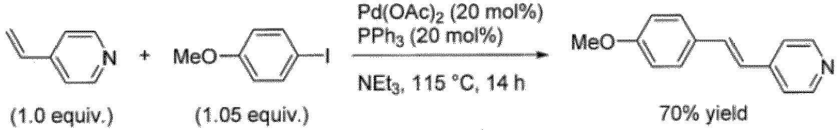

In an argon atmosphere, palladium acetate (44.9 mg, 0.2 mmol), triphenylphosphine (52.5 mg, 0.2 mmol), 4-vinylpyridine (105.1 mg, 1.0 mmol), and 4-iodoanisole (245.7 mg, 1.05 mmol) were added to triethylamine (1.5 mL). The obtained mixture was heated and stirred at 115°C for 14 hours. After cooling of the reaction mixture to room temperature, the mixture was filtered through silica gel using methylene chloride. The filtrate was concentrated under reduced pressure to obtain a crude product. The obtained crude product was purified by silica gel chromatography (using hexane:ethyl acetate:triethylamine = 50:50:1 as a developing solvent) and further recrystallized with a mixed solvent of methylene chloride and hexane to obtain the target compound as a pale yellow solid (147.9 mg, 70%).

### [Example 2] 4-([1,1'-Biphenyl]-4-yl)pyridine

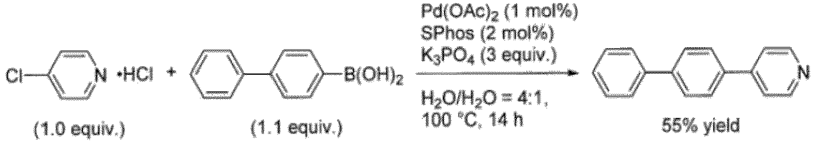

In an argon atmosphere, palladium acetate (11.2 mg, 0.05 mmol), 2-dicyclohexylphosphino-2',6'-dimethoxybiphenyl (41.0 mg, 0.1 mmol), 4-chloropyridine hydrochloride (750 mg, 5.0 mmol), 4-biphenylboronic acid (1.09 g, 5.5 mmol), and tripotassium phosphate (3.18 g, 15.0 mmol) were added to a mixture of water (20 mL) and acetonitrile (5 mL). The obtained mixture was heated and stirred at 100°C for 14 hours. After cooling of the reaction mixture to room temperature, the aqueous layer was subjected to extraction three times in total, using 30 mL of methylene chloride per one extraction. The combined organic layer was washed with saturated saline and dried over sodium sulfate. After removal of the solid by filtration, the filtrate was concentrated under reduced pressure to obtain a crude product. The obtained crude product was purified by silica gel chromatography (using hexane:ethyl acetate = 1:1 and then hexane:ethyl acetate:methylene chloride = 3:7:2 as a developing solvent) and further recrystallized with a mixed solvent of methylene chloride and hexane. The obtained solid was dissolved in methylene chloride, followed by extraction with 1 N hydrochloric acid. The combined aqueous layer was rendered basic with sodium hydroxide, and the resulting aqueous layer was subjected to extraction with methylene chloride. The combined organic layer was concentrated under reduced pressure and dried in vacuum to obtain the target compound as a white solid (630 mg, 55%) .

### [Example 3] 9-Methoxyacridine

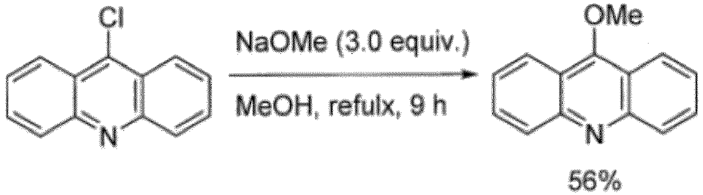

In an argon atmosphere, 9-chloroacridine (641 mg, 3.00 mmol) was added to and dissolved in methanol (10 mL). To the solution, a solution of sodium methoxide in methanol (5.0 M, 1.8 mL, 9.0 mmol) was further added. The obtained mixture was stirred by heating to reflux for 9 hours. After cooling of the reaction mixture to room temperature, the mixture was concentrated under reduced pressure. The obtained residue was separated into an organic layer and an aqueous layer by the addition of water (30 mL) and methylene chloride (30 mL). The aqueous layer was subjected to extraction three times in total, using 30 mL of methylene chloride per one extraction. The combined organic layer was washed with saturated saline and dried over sodium sulfate. After removal of the solid by filtration, the filtrate was concentrated under reduced pressure to obtain a crude product. The obtained crude product was purified by silica gel chromatography (using methylene chloride:methanol = 20:1 as a developing solvent) to obtain the target compound as a pale yellow solid (351 mg, 56%) .

### [Example 4] 2-(4-(Methoxymethoxy)phenyl)acetonitrile

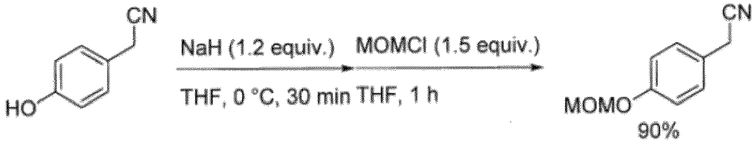

In an argon atmosphere, 4-hydroxybenzyl cyanide (4.0 g, 30 mmol) was added to tetrahydrofuran (60 mL), and sodium hydride (powder dispersed in 60% liquid paraffin, 1.44 g, 36 mmol) was further added thereto at 0°C. The obtained mixture was stirred at room temperature for 30 minutes. To the reaction mixture, chloromethyl methyl ether (3.39 mL, 45.0 mmol) was added at 0°C, and the mixture was stirred at room temperature for 1 hour. Water (20 mL) was added to the reaction mixture to terminate the reaction. After separation into an organic layer and an aqueous layer, the aqueous layer was subjected to extraction three times in total, using 20 mL of methylene chloride per one extraction. The combined organic layer was washed with saturated saline and dried over sodium sulfate. After removal of the solid by filtration, the filtrate was concentrated under reduced pressure to obtain a crude product. The obtained crude product was purified by silica gel chromatography (using hexane:ethyl acetate = 100:1, subsequently hexane:ethyl acetate = 5:1, subsequently hexane:ethyl acetate = 4:1, and subsequently hexane:ethyl acetate = 2:1 as a developing solvent) to obtain the target compound as a colorless liquid (4.80 g, 90%).

### [Example 5] 2,3-Bis(4-(methoxymethoxy)phenyl)fumaronitrile

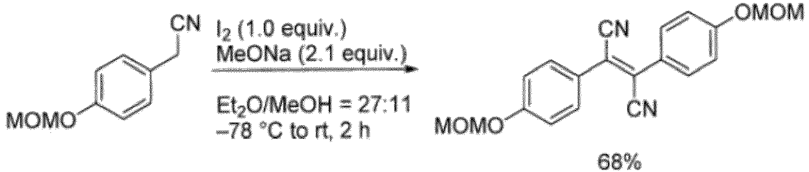

In an argon atmosphere, a solution of sodium methoxide in methanol (5.0 M, 11.3 mL, 56.7 mmol) was added to a solution of 2-(4-(methoxymethoxy)phenyl)acetonitrile (4.80 g, 27.0 mmol) and iodine (6.86 g, 27 mmol) added to diethyl ether (27 mL) at -78°C over 5 minutes. The obtained mixture was stirred at room temperature for 2 hours. The reaction mixture was filtered through filter paper. The obtained solid was washed with a mixed solution of cold water and methanol (1:1) and subsequently washed with a small amount of diethyl ether to obtain the target compound as a pale yellow powder (3.23 g, 68%).

### [Example 6] 2,3-Bis(4-hydroxyphenyl)fumaronitrile

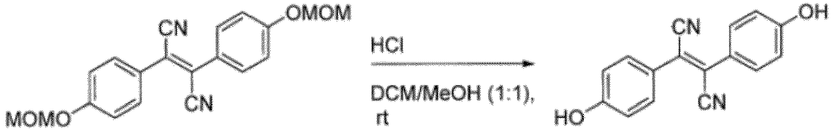

In an argon atmosphere, 2,3-bis(4-(methoxymethoxy)phenyl)fumaronitrile (2.8 g, 8 mmol) was added to and dissolved in methylene chloride (24 mL). A solution of hydrochloric acid in methanol (5%, 24 mL) was further added to the solution. The obtained mixture was stirred at room temperature for 22 hours. The reaction mixture was filtered through filter paper. The obtained solid was washed with diethyl ether to obtain the target compound as an orange solid (308 mg). The solid deposited in the filtrate was filtered through filter paper to further obtain the target compound as an orange solid (866 mg). The filtrate was concentrated under reduced pressure, and the deposited solid was washed with a small amount of chloroform to further obtain the target compound as an orange solid (804 mg). The total of the obtained solids was 1.98 g, and the yield was 94%.

### [Example 7]

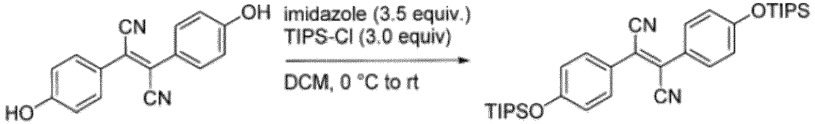

In an argon atmosphere, 2,3-bis(4-hydroxyphenyl)fumaronitrile (1.05 g, 4 mmol), imidazole (953 mg, 14 mmol), and triisopropylsilyl chloride (2.31 g, 12 mmol) were added to methylene chloride (40 mL) at 0°C. The obtained mixture was stirred at room temperature for 8 hours. Water was added to the reaction mixture to terminate the reaction, followed by separation into an organic layer and an aqueous layer. The aqueous layer was subjected to extraction three times in total, using 30 mL of methylene chloride per one extraction. The combined organic layer was washed with saturated saline and dried over sodium sulfate. After removal of the solid by filtration, the filtrate was concentrated under reduced pressure to obtain a crude product. The obtained crude product was filtered through silica gel and recrystallized with a mixed solvent of methylene chloride and methanol to obtain the target compound as a light green solid (1.82 g, 79%).

### [Example 8]

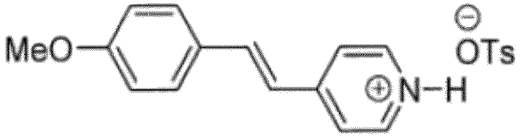

In the atmosphere, (E)-4-(4-methoxystyryl)pyridine (21.1 mg, 0.1 mmol) and p-toluenesulfonic acid monohydrate (19.0 mg, 0.1 mmol) were added to and dissolved in methylene chloride. The obtained mixture was stirred at room temperature for 5 minutes. The solvent in the reaction mixture was distilled off under reduced pressure to obtain the target compound as a yellow solid.

### [Example 9]

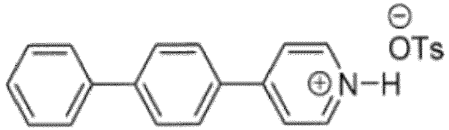

In the atmosphere, 4-([1,1'-biphenyl]-4-yl)pyridine (23.1 mg, 0.1 mmol) and p-toluenesulfonic acid monohydrate (19.0 mg, 0.1 mmol) were added to and dissolved in methylene chloride. The obtained mixture was stirred at room temperature for 5 minutes. The solvent in the reaction mixture was distilled off under reduced pressure to obtain the target compound as a yellow solid.

### [Example 10]

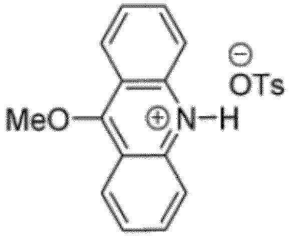

In the atmosphere, 9-methoxyacridine (20.9 mg, 0.1 mmol) and p-toluenesulfonic acid monohydrate (19.0 mg, 0.1 mmol) were added to and dissolved in methylene chloride. The obtained mixture was stirred at room temperature for 5 minutes. The solvent in the reaction mixture was distilled off under reduced pressure to obtain the target compound as a yellow solid.

### [Example 11]

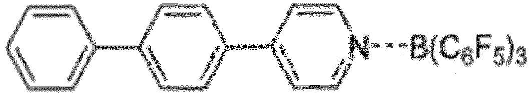

In the atmosphere, 4-([1,1'-biphenyl]-4-yl)pyridine (23.1 mg, 0.1 mmol) and tris(pentafluorophenyl)borane (51.2 mg, 0.1 mmol) were added to and dissolved in methylene chloride. The obtained mixture was stirred at room temperature for 5 minutes. The solvent in the reaction mixture was distilled off under reduced pressure to obtain the target compound as a white solid.

### [Example 12]

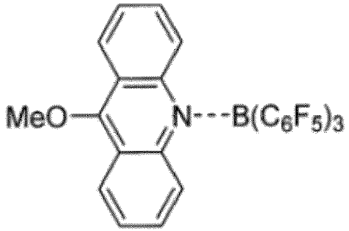

In the atmosphere, 9-methoxyacridine (20.9 mg, 0.1 mmol) and tris(pentafluorophenyl)borane (51.2 mg, 0.1 mmol) were added to and dissolved in methylene chloride. The obtained mixture was stirred at room temperature for 5 minutes. The solvent in the reaction mixture was distilled off under reduced pressure to obtain the target compound as a yellow solid.

### [Example 13]

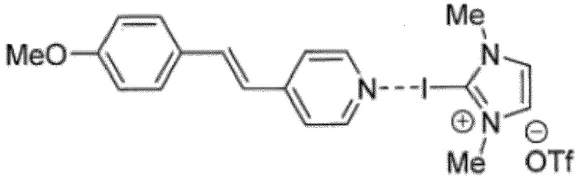

In the atmosphere, (E)-4-(4-methoxystyryl)pyridine (21.1 mg, 0.1 mmol) and 2-iodo-1,3-dimethyl-1H-imidazol-3-ium trifluoromethanesulfonate (37.2 mg, 0.1 mmol) were added to and dissolved in methylene chloride. The obtained mixture was stirred at room temperature for 5 minutes. The solvent in the reaction mixture was distilled off under reduced pressure to obtain the target compound as a yellow solid.

### [Example 14]

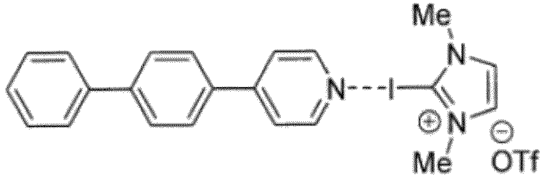

In the atmosphere, 4-([1,1'-biphenyl]-4-yl)pyridine (23.1 mg, 0.1 mmol) and 2-iodo-1,3-dimethyl-1H-imidazol-3-ium trifluoromethanesulfonate (37.2 mg, 0.1 mmol) were added to and dissolved in methylene chloride. The obtained mixture was stirred at room temperature for 5 minutes. The solvent in the reaction mixture was distilled off under reduced pressure to obtain the target compound as a white solid.

### [Example 15]

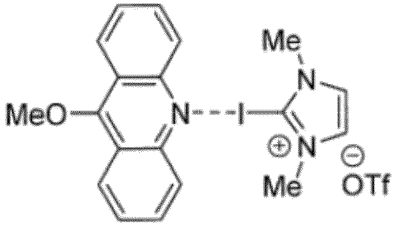

In the atmosphere, 9-methoxyacridine (20.9 mg, 0.1 mmol) and 2-iodo-1,3-dimethyl-1H-imidazol-3-ium trifluoromethanesulfonate (37.2 mg, 0.1 mmol) were added to and dissolved in methylene chloride. The obtained mixture was stirred at room temperature for 5 minutes. The solvent in the reaction mixture was distilled off under reduced pressure to obtain the target compound as a yellow solid.

### [Example 16]

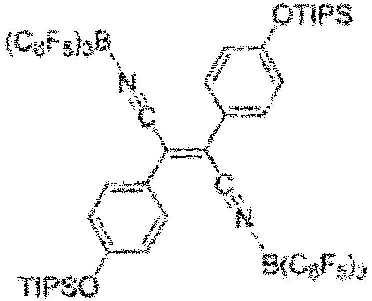

In the atmosphere, 2,3-bis(4-((triisopropylsilyl)oxy)phenyl)fumaronitrile (28.7 mg, 0.05 mmol) and tris(pentafluorophenyl)borane (51.2 mg, 0.1 mmol) were added to and dissolved in methylene chloride. The obtained mixture was stirred at room temperature for 5 minutes. The solvent in the reaction mixture was distilled off under reduced pressure to obtain the target compound as a yellow solid.

### [Example 17]

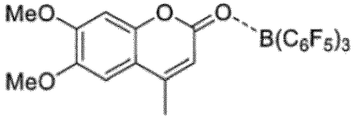

In the atmosphere, 6,7-dimethoxy-4-methylcoumarin (20.6 mg, 0.1 mmol) and tris(pentafluorophenyl)borane (51.2 mg, 0.1 mmol) were added to and dissolved in acetone and methylene chloride. The obtained mixture was stirred at room temperature for 5 minutes. The solvent in the reaction mixture was distilled off under reduced pressure to obtain the target compound as a yellow solid.

### [Example 18]

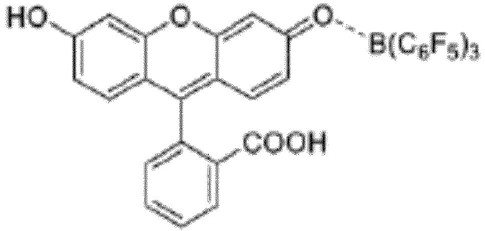

In the atmosphere, fluorescein (33.2 mg, 0.1 mmol) and tris(pentafluorophenyl)borane (51.2 mg, 0.1 mmol) were added to and dissolved in acetone and methylene chloride. The obtained mixture was stirred at room temperature for 5 minutes. The solvent in the reaction mixture was distilled off under reduced pressure to obtain the target compound as a yellow solid.

### [Example 19]

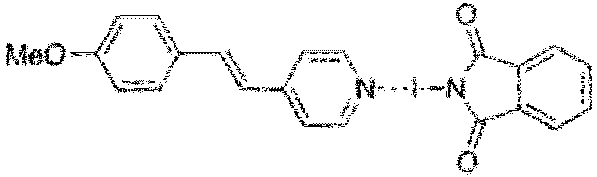

(E)-4-(4-Methoxystyryl)pyridine (21.1 mg, 0.1 mmol) and 2-iodoisoindoline-1,3-dione (27.3 mg, 0.1 mmol) were added to and dissolved in a mixed solvent of acetone and methylene chloride. The obtained mixture was stirred at room temperature for 5 minutes. The solvent in the reaction mixture was distilled off under reduced pressure to obtain the target compound as a grayish white solid.

### [Example 20]

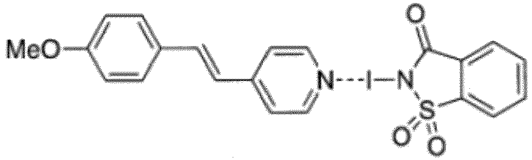

In the atmosphere, (E)-4-(4-methoxystyryl)pyridine (21.1 mg, 0.1 mmol) and 2-iodobenz[d]isothiazol-3(2H)-one 1,1-dioxide (30.9 mg, 0.1 mmol) were added to and dissolved in a mixed solvent of acetone and methylene chloride. The obtained mixture was stirred at room temperature for 5 minutes. The solvent in the reaction mixture was distilled off under reduced pressure to obtain the target compound as a yellow solid.

### [Example 21]

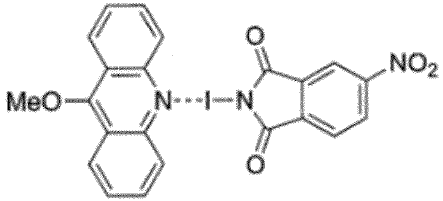

In the atmosphere, 9-methoxyacridine (20.9 mg, 0.1 mmol) and 2-iodo-5-nitroisoindoline-1,3-dione (31.8 mg, 0.1 mmol) were added to and dissolved in acetone and methylene chloride. The obtained mixture was stirred at room temperature for 5 minutes. The solvent in the reaction mixture was distilled off under reduced pressure to obtain the target compound as a yellow solid.

### [Example 22]

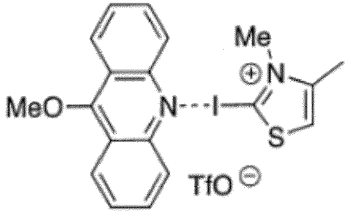

In the atmosphere, 9-methoxyacridine (20.9 mg, 0.1 mmol) and 2-iodo-3,4-dimethylthiazol-3-inium trifluorosulfonate (39.9 mg, 0.1 mmol) were added to and dissolved in acetone and methylene chloride. The obtained mixture was stirred at room temperature for 5 minutes. The solvent in the reaction mixture was distilled off under reduced pressure to obtain the target compound as a yellow solid.

### [Example 23]

The compound obtained in Example 1 was subjected to a measurement of a fluorescence spectrum and a fluorescence quantum yield.

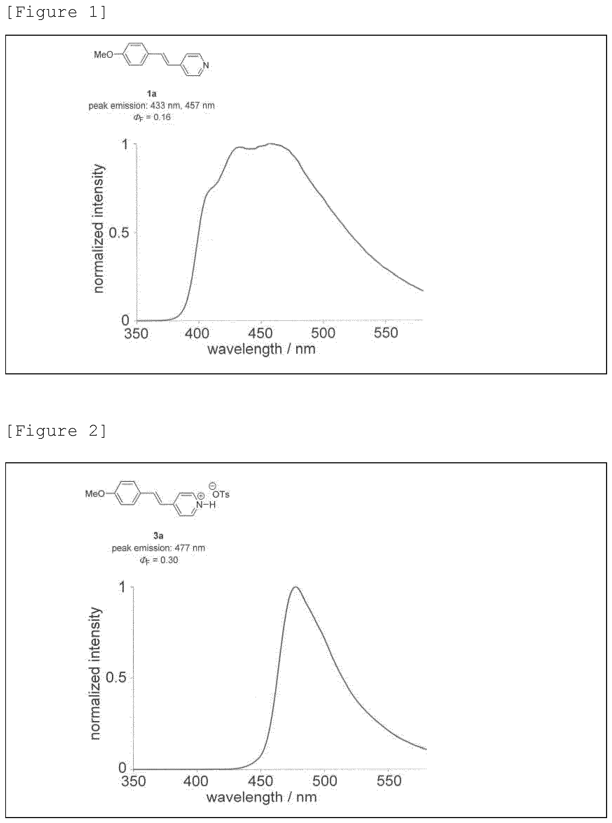

The fluorescence spectrum measurement was performed using Hitachi F-7000. The fluorescence quantum yield measurement was performed using Absolute PL Quantum Yield Spectrometer C11347, Quantaurus-QY (Hamamatsu Photonics K.K.). The fluorescence lifetime was measured using Quantaurus-Tau C11367-24 (Hamamatsu Photonics K.K.). The emission spectrum in a solid state using a wavelength of 300 nm as an excitation wavelength is shown in Figure 1.

### [Example 24]

The same measurements as in Example 23 were performed on the compound obtained in Example 8. The emission spectrum in a solid state using a wavelength of 300 nm as an excitation wavelength is shown in Figure 2.

### [Example 25]

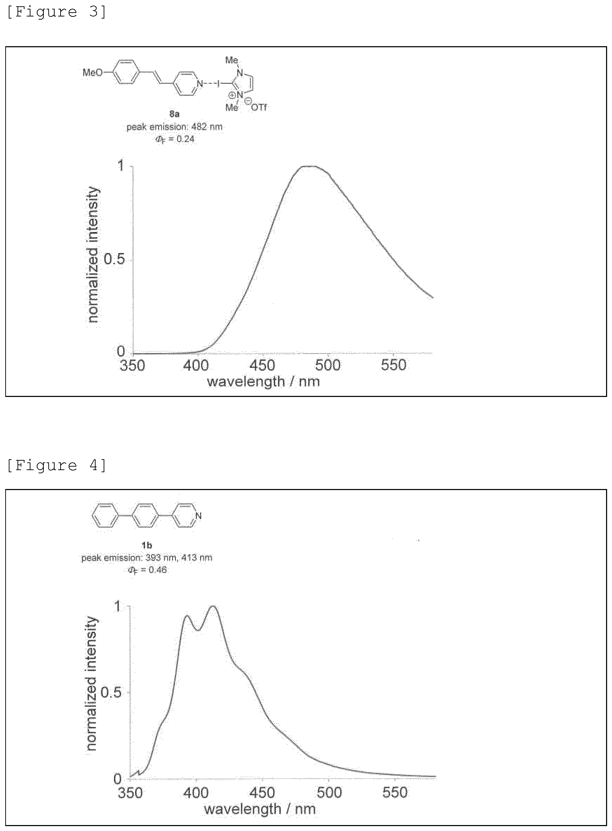

The same measurements as in Example 23 were performed on the compound obtained in Example 13. The emission spectrum in a solid state using a wavelength of 300 nm as an excitation wavelength is shown in Figure 3.

### [Example 26]

The same measurements as in Example 23 were performed on the compound obtained in Example 2. The emission spectrum in a solid state using a wavelength of 300 nm as an excitation wavelength is shown in Figure 4.

### [Example 27]

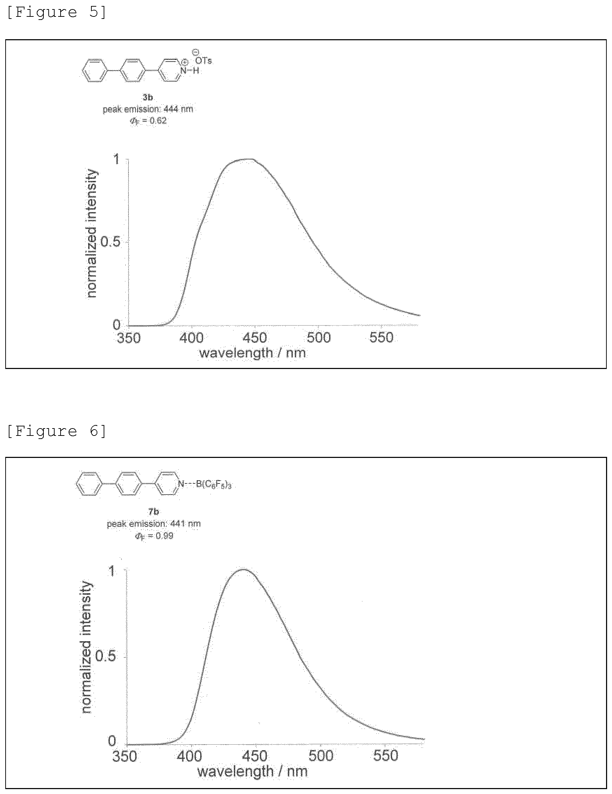

The same measurements as in Example 23 were performed on the compound obtained in Example 9. The emission spectrum in a solid state using a wavelength of 300 nm as an excitation wavelength is shown in Figure 5.

### [Example 28]

The same measurements as in Example 23 were performed on the compound obtained in Example 11. The emission spectrum in a solid state using a wavelength of 300 nm as an excitation wavelength is shown in Figure 6.

### [Example 29]

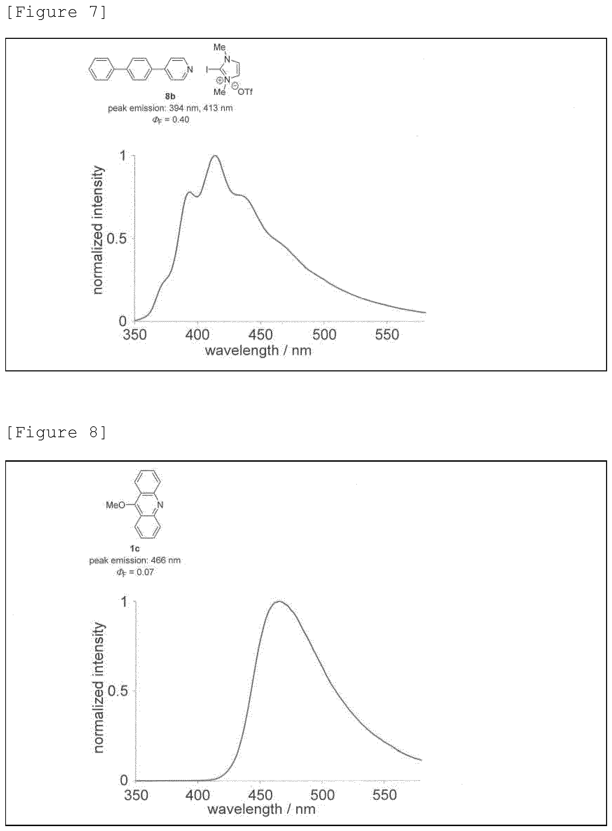

The same measurements as in Example 23 were performed on the compound obtained in Example 14. The emission spectrum in a solid state using a wavelength of 300 nm as an excitation wavelength is shown in Figure 7.

### [Example 30]

The same measurements as in Example 23 were performed on the compound obtained in Example 3. The emission spectrum in a solid state using a wavelength of 300 nm as an excitation wavelength is shown in Figure 8.

### [Example 31]

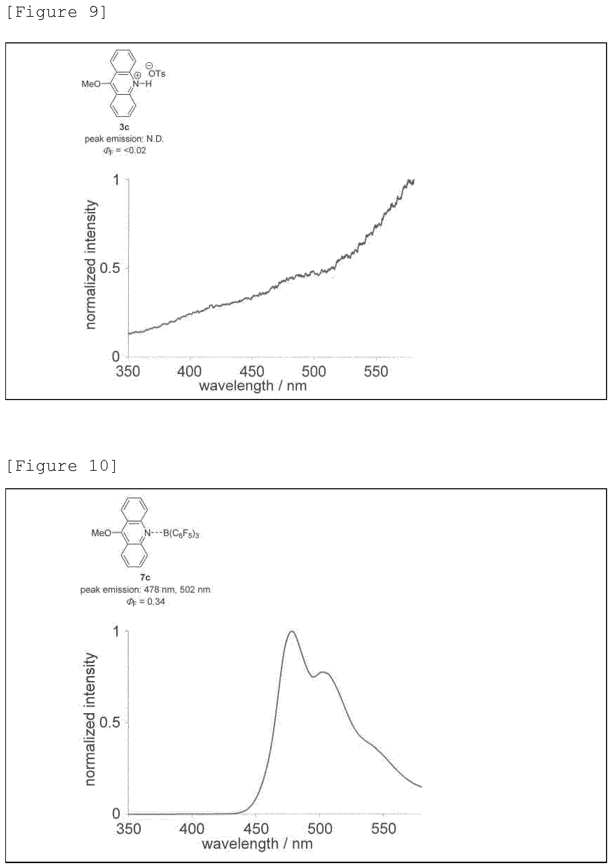

The same measurements as in Example 23 were performed on the compound obtained in Example 10. The emission spectrum in a solid state using a wavelength of 300 nm as an excitation wavelength is shown in Figure 9.

### [Example 32]

The same measurements as in Example 23 were performed on the compound obtained in Example 12. The emission spectrum in a solid state using a wavelength of 300 nm as an excitation wavelength is shown in Figure 10.

### [Example 33]

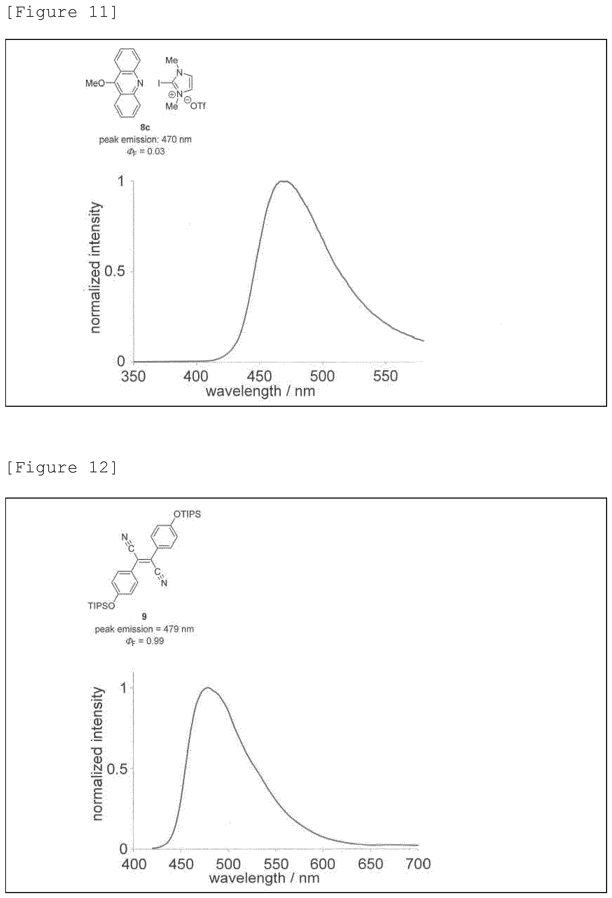

The same measurements as in Example 23 were performed on the compound obtained in Example 15. The emission spectrum in a solid state using a wavelength of 300 nm as an excitation wavelength is shown in Figure 11.

### [Example 34]

The same measurements as in Example 23 were performed on the compound obtained in Example 7. The emission spectrum in a solid state using a wavelength of 400 nm as an excitation wavelength is shown in Figure 12.

### [Example 35]

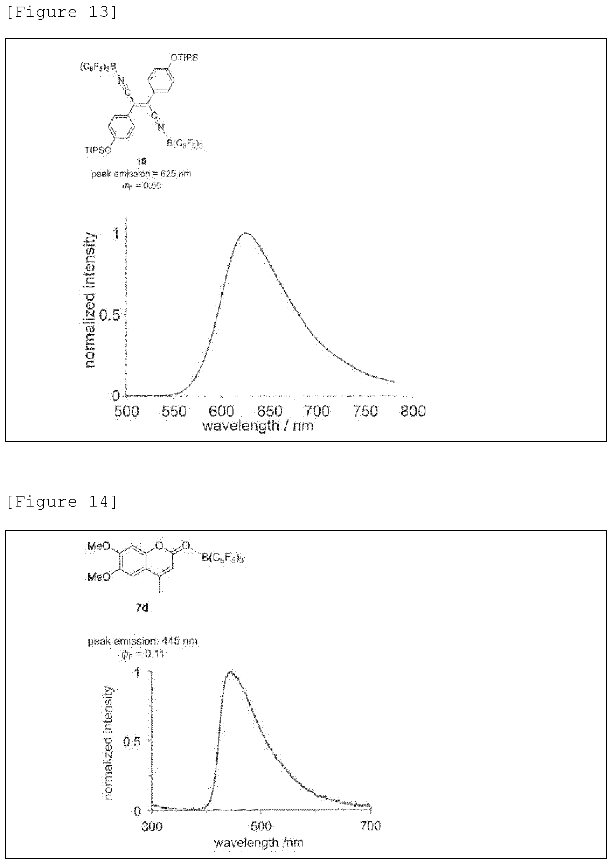

The same measurements as in Example 23 were performed on the compound obtained in Example 16. The emission spectrum in a solid state using a wavelength of 400 nm as an excitation wavelength is shown in Figure 13.

### [Example 36]

The same measurements as in Example 23 were performed on the compound obtained in Example 17. The emission spectrum in a solid state using a wavelength of 280 nm as an excitation wavelength is shown in Figure 14.

### [Example 37]

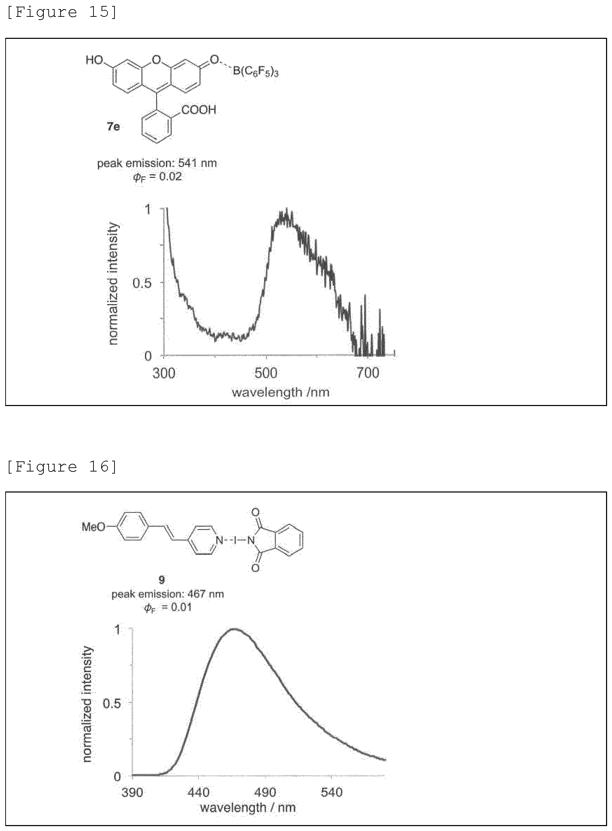

The same measurements as in Example 23 were performed on the compound obtained in Example 18. The emission spectrum in a solid state using a wavelength of 280 nm as an excitation wavelength is shown in Figure 15.

### [Example 38]

The same measurements as in Example 23 were performed on the compound obtained in Example 19. The emission spectrum in a solid state using a wavelength of 365 nm as an excitation wavelength is shown in Figure 16.

### [Example 39]

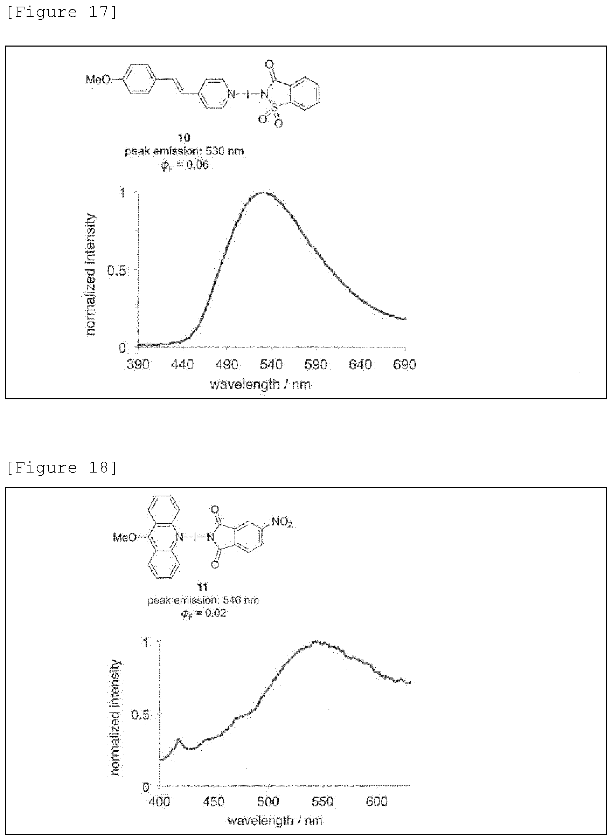

The same measurements as in Example 23 were performed on the compound obtained in Example 20. The emission spectrum in a solid state using a wavelength of 365 nm as an excitation wavelength is shown in Figure 17.

### [Example 40]

The same measurements as in Example 23 were performed on the compound obtained in Example 21. The emission spectrum in a solid state using a wavelength of 365 nm as an excitation wavelength is shown in Figure 18.

### [Example 41]

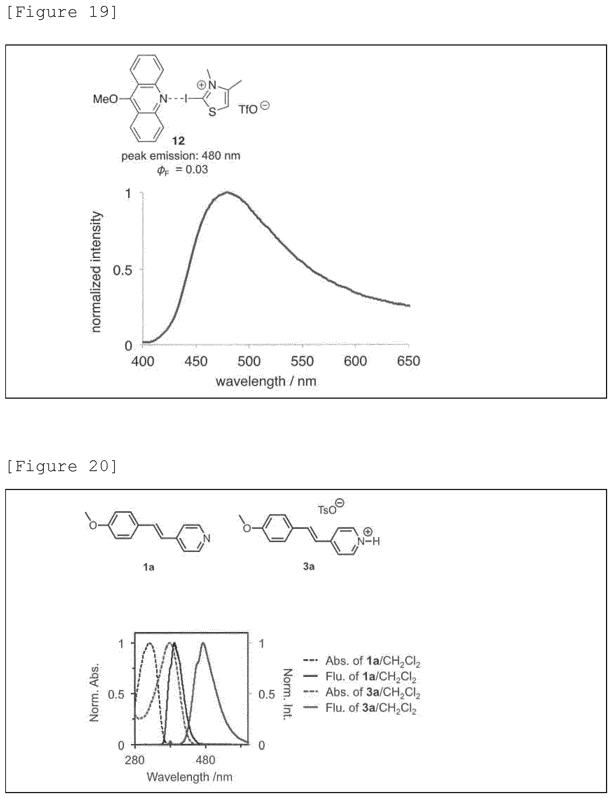

The same measurements as in Example 23 were performed on the compound obtained in Example 22. The emission spectrum in a solid state using a wavelength of 365 nm as an excitation wavelength is shown in Figure 19.

### [Examples 42 to 83]

The following complexes 42 to 83 were produced in the same way as in Examples described above.

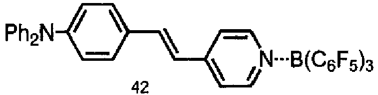

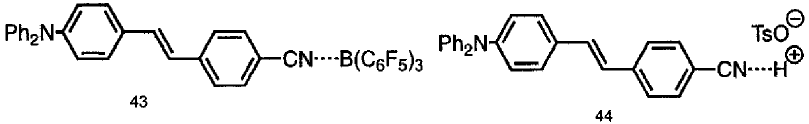

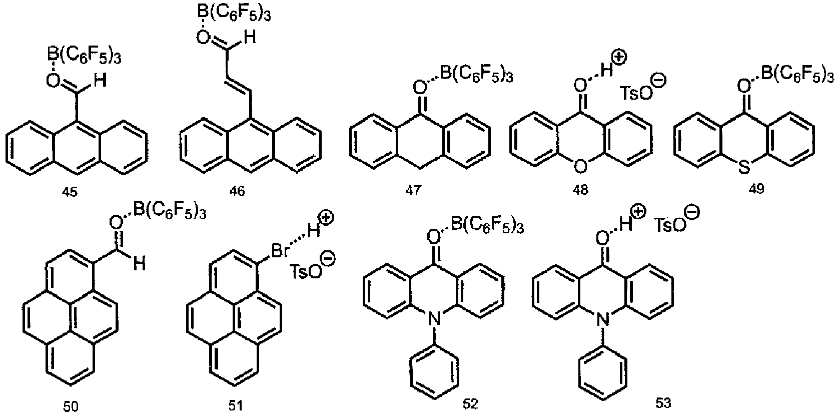

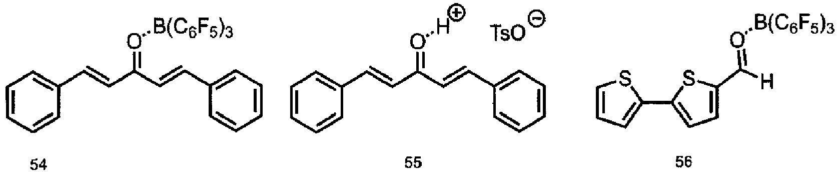

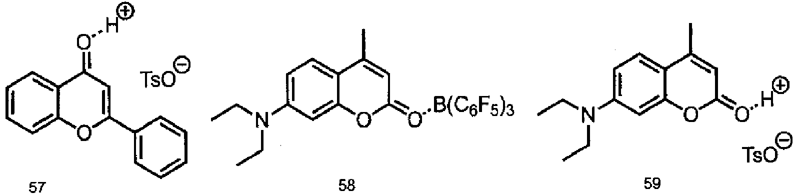

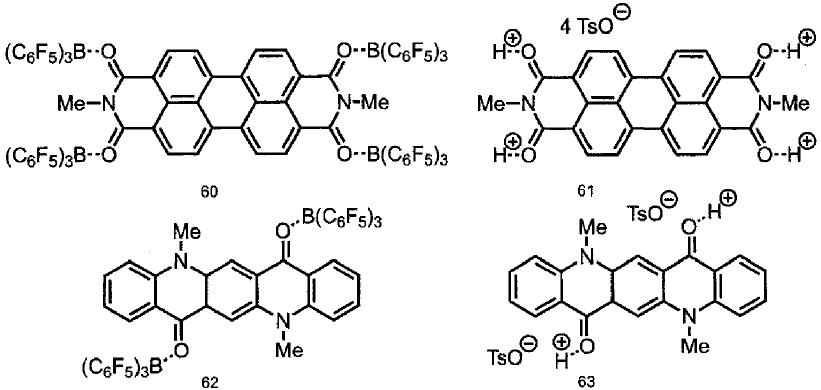

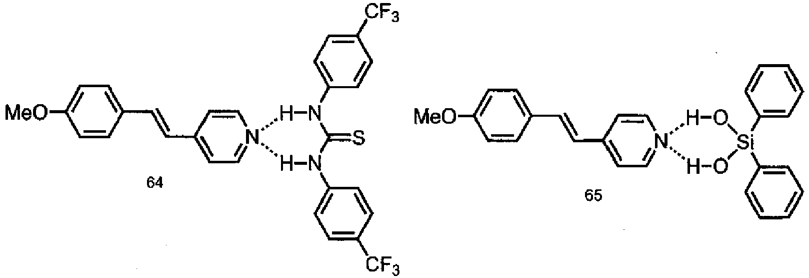

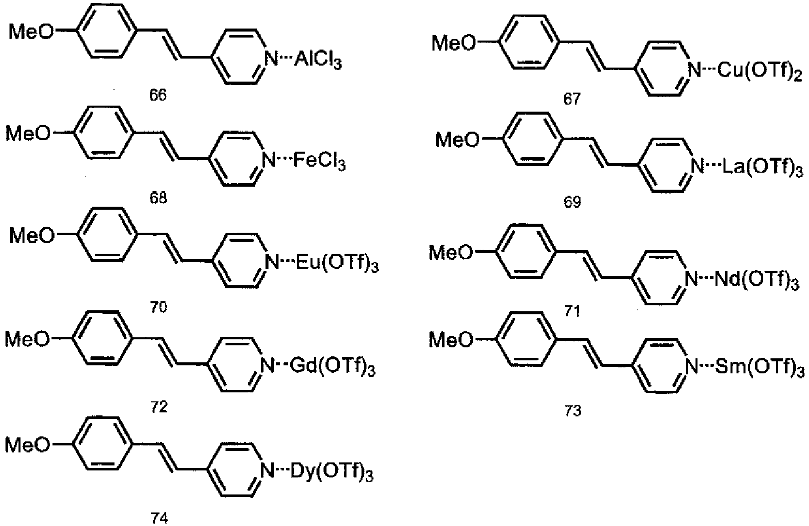

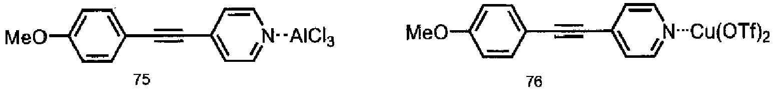

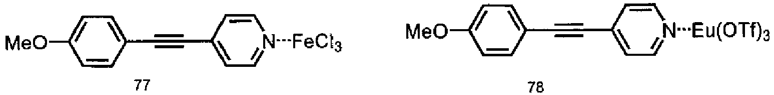

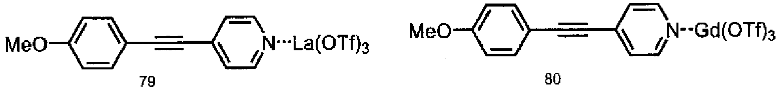

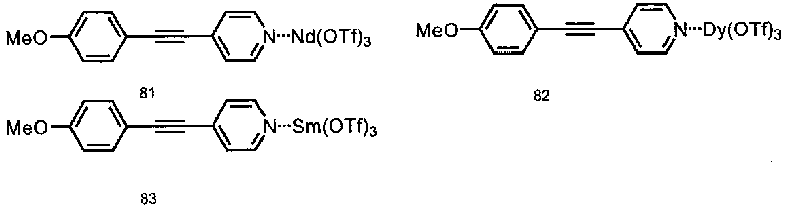

The emission spectra (peak wavelengths), fluorescence quantum yields, and fluorescence lifetimes of the complexes obtained in Examples described above are summarized in Table 1. The notation within the parentheses in the column "Lifetime" indicating the fluorescence lifetime represents the lifetime of the two-component system in a fluorescence decay curve. As is evident, the obtained complexes exhibit a very high fluorescence quantum yield. In the table, the term "starting material" denotes a compound before complex formation.

**[Table 1]**

| Example No. of complex | Fluorescence (nm) | Quantum yield | Lifetime τ (ns) |
|---|---|---|---|
| Example 1 (Starting material of Example 8,13) | 433,457 | 0.16 | 1.12 (0.66, 1.92) |
| Example 8 | 477 | 0.30 | 0.96 (0.61, 1.22) |
| Example 13 | 482 | 0.24 | 6.1 (2.2, 7.1) |
| Example 3 (Starting material of Example 10,12,15) | 466 | 0.07 | 11.0 (3.8, 12.8) |
| Example 12 | 478, 502 | 0.34 | 15.8 (7.3, 18.5) |
| Example 10 | N.D. | <0.02 | --- |
| Example 15 | 470 | 0.03 | --- |
| Example 2 (Starting material of Example 9,11,14) | 393, 413 | 0.46 | 1.41 (1.2, 4.4) |
| Example 11 | 441 | 0.99 | 5.94 (2.4, 8.4) |
| Example 9 | 444 | 0.62 | 1.86 (1.6, 7.2) |
| Example 14 | 394, 413 | 0.4 | --- |
| Example 7 (Starting material of Example 16) | 479 | 0.99 | 3.34 |
| Example 16 | 625 | 0.5 | 6.05 |
| Example 17 | 445 | 0.11 | --- |
| Example 18 | 541 | 0.02 | --- |
| Example 19 | 460 | 0.01 | 0.24 (0.19, 0.55) |
| Example 20 | 530 | 0.06 | 1.10 (0.20, 2.10) |
| Example 21 | 546 | 0.02 | 3.83 (0.96, 6.85) |
| Example 22 | 480 | 0.03 | 0.38 (0.30, 0.77) |
| (Starting material of 42) | 588 | 0.01 | |
| 42 | 637 | 0.14 | --- |
| (Starting material of 43,44) | 500 | 0.09 | --- |
| 43 | 637 | 0.02 | --- |
| 44 | 587 | 0.08 | --- |
| (Starting material of 45) | 536 | 0.19 | --- |
| 45 | 745 | <0.01 | --- |
| (Starting material of 46) | 557 | 0.04 | --- |
| 46 | 783 | <0.01 | --- |
| (Starting material of 47) | 395 | <0.01 | --- |
| 47 | 500 | <0.01 | --- |
| 48 | 421 | 0.01 | --- |
| (Starting material of 49) | 546 | 0.02 | --- |
| 49 | 540 | <0.01 | --- |
| (Starting material of 50) | 543 | 0.05 | --- |
| 50 | 675 | 0.07 | --- |
| (Starting material of 51) | 432 | 0.02 | --- |
| 51 | 423 | 0.01 | --- |
| (Starting material of 51,53) | 471 | 0.07 | --- |
| 52 | 517 | 0.02 | --- |
| 53 | 486 | 0.01 | --- |
| (Starting material of 54,55) | 513 | <0.01 | --- |
| 54 | 560 | 0.07 | --- |
| 55 | 745 | <0.01 | --- |
| (Starting material of 56) | 486 | 0.02 | --- |
| 56 | 498 | 0.02 | --- |
| (Starting material of 57) | <300 | --- | --- |
| 57 | 471 | 0.08 | --- |
| (Starting material of 58,59) | 444 | 0.17 | --- |
| 58 | 482 | 0.73 | --- |
| 59 | 493 | 0.03 | --- |
| (Starting material of 60,61) | 755 | <0.01 | --- |
| 60 | 796 | <0.01 | --- |
| 61 | 789 | <0.01 | --- |
| (Starting material of 62,63) | 669 | 0.02 | --- |
| 62 | 824 | <0.01 | --- |
| 63 | 824 | <0.01 | --- |
| (Starting material of 64,65) | 433,457 | 0.16 | --- |
| 64 | 560 | 0.06 | --- |
| 65 | 479 | 0.01 | --- |

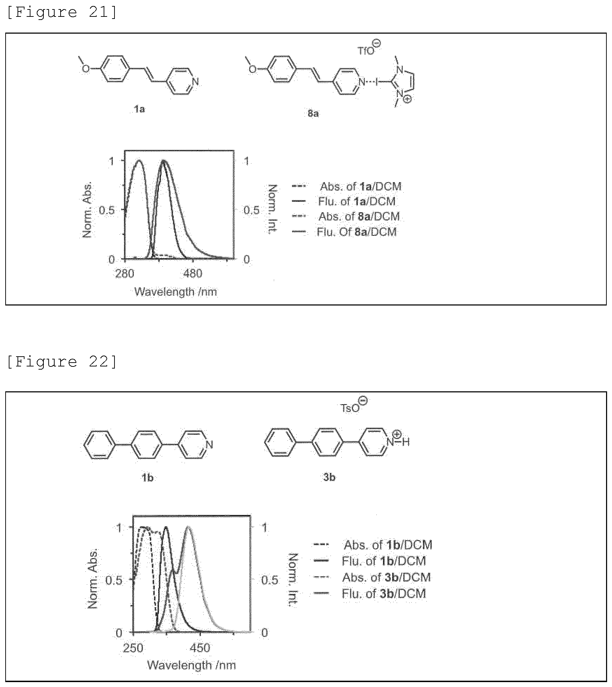

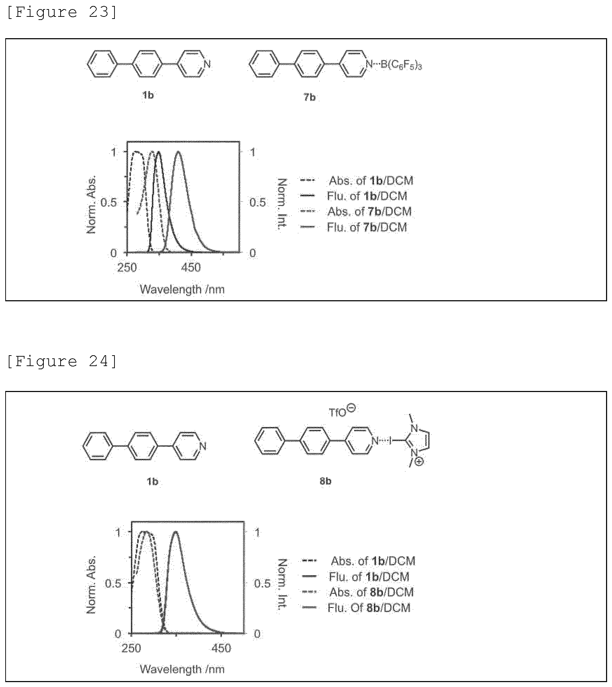

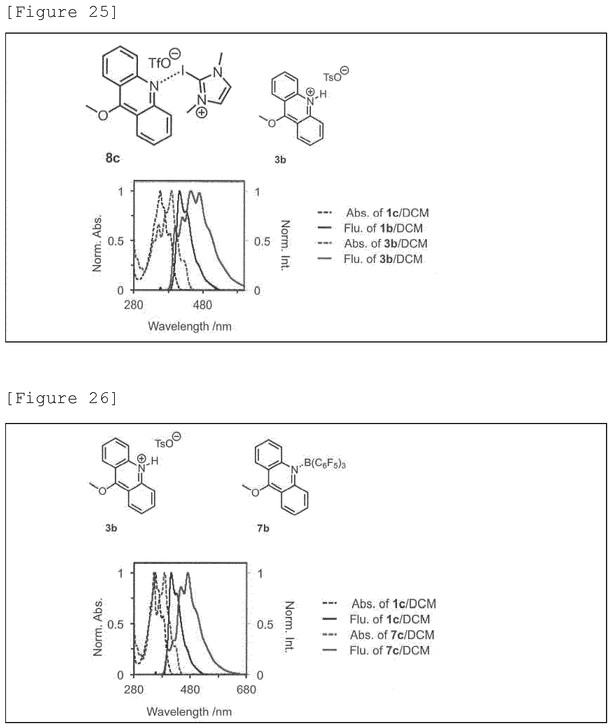

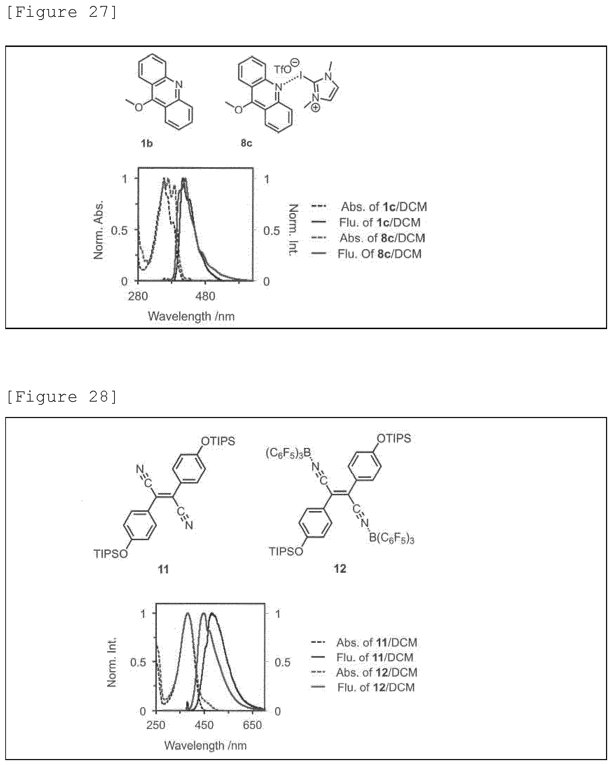

The emission spectra of the complexes obtained in Examples described above were measured in a dichloromethane (DCM) or acetone solution. The results are shown in Figures 20 to 28 and Table 2.

**[Table 2]**

| Example No. of complex | Absorbance εₘₐₓ (10⁴ M⁻¹·cm⁻¹) | Maximum absorption wavelength λ_{abs, max} (nm) | Maximum fluorescence wavelength λ_{em, max} (nm) | Excitation wavelength λₑₓ(nm) | Stokes shift (cm⁻¹) | Stokes shift | Ratio |
|---|---|---|---|---|---|---|---|
| | | | | | | (nm) | % |
| | | | | | | | |
| Example 1 (Starting material of Example 8,13) | 3.20 | 320 | 393 | 320 | 5805 | 73 | 22.8 |
| Example 8 | 2.39 | 380 | 473 | 380 | 5174 | 93 | 24.5 |
| Example 13 | 3.37 | 321 | 397 | 321 | 5964 | 76 | 23.7 |
| Example 2 (Starting material of Example 9,11,14) | 3.19 | 273 | 347 | 273 | 7812 | 74 | 27.1 |
| Example 9 | 1.73 | 324 | 418 | 324 | 6941 | 94 | 29.0 |
| Example 11 | 1.85 | 327 | 409 | 327 | 6131 | 82 | 25.1 |
| Example 14 | 2.39 | 284 | 348 | 284 | 6476 | 64 | 22.5 |
| Example 3 (Starting material of Example10,12,15) | 0.857 | 357 | 412 | 357 | 3739 | 55 | 15.4 |
| Example 10 | 0.607 | 389 | 441 | 389 | 3031 | 52 | 13.4 |
| Example 12 | 0.807 | 389 | 471 | 389 | 4476 | 82 | 21.1 |
| Example 15 | 0.571 | 369 | 420 | 369 | 3291 | 51 | 13.8 |
| Example 7 (Starting material of Example16) | 2.17 | 380 | 484 | 380 | 5655 | 104 | 27.4 |
| Example 16 | 1.86 | 381 | 448 | 381 | 3925 | 67 | 17.6 |
| Example 17 | - | <340 | 322 | 280 | - | - | - |
| Example 18 | 2.54 | 439 | 472 | 439 | 1593 | 33 | 7.5 |
| Example 19 | 3.66 | 335 | 441 | 335 | 7175 | 106 | 31.6 |
| Example 20 | 3.66 | 355 | 471 | 355 | 6938 | 116 | 32.7 |
| Example 21 | 0.444 | 368 | 401 | 335 | 2236 | 33 | 9.0 |
| Example 22 | 0.962 | 355 | 422 | 355 | 4473 | 67 | 18.9 |
| (Starting material of 42) | 1.80 | 382 | 515 | 382 | 6760 | 133 | 34.8 |
| 42 | 2.38 | 463 | 517 | 463 | 2256 | 54 | 11.7 |
| (Starting material of 43,44) | 2.39 | 394 | 515 | 394 | 5963 | 121 | 30.7 |
| 43 | 2.81 | 398 | 517 | 398 | 5783 | 119 | 29.9 |
| 44 | 1.94 | 382 | 515 | 382 | 6761 | 133 | 34.8 |
| (Starting material of 45) | 7.35 | 403 | 484 | 400 | 4339 | 81 | 20.1 |
| 45 | 0.03 | 499 | 558 | 500 | 2079 | 59 | 11.8 |
| (Starting material of 49) | 7.07 | 381 | 411 | 381 | 1916 | 30 | 7.9 |
| 49 | 0.01 | 459 | 516 | 460 | 2407 | 57 | 12.4 |
| (Starting material of 50) | 2.30 | 400 | 423 | 400 | 1359 | 23 | 5.8 |
| 50 | 0.04 | 489 | 522 | 490 | 1293 | 33 | 6.7 |
| (Starting material of 52,53) | 2.42 | 395 | 406 | 395 | 686 | 11 | 2.8 |
| 52 | [overflow] | 395 | 445,466 | 395 | 3857 | 50,71 | 12.7,18.0 |
| (Starting material of 56) | 2.29 | 354 | 423 | 355 | 4608 | 69 | 19.5 |
| 56 | 0.18 | 440 | 479 | 440 | 1850 | 39 | 8.9 |
| (Starting material of 58,59) | 5.53 | 372 | 425 | 370 | 3352 | 53 | 14.2 |
| 58 | 0.37 | 433 | 479 | 430 | 2218 | 46 | 10.6 |

Each complex exhibited almost the same fluorescence wavelength when measured in a solid state (Table 1) and when measured in a solution (Table 2), though the numeric values were not in complete agreement. This does not deny use of measurement in a solution instead of difficult measurement in a solid state. However, the numeric values were not in complete agreement probably because a solid and a liquid differ in the contribution of the electron acceptor X to the HOMO and LUMO electron levels of the pi-conjugated molecule W. These results further indicate that the fluorescence wavelength can be adjusted by the type of the electron acceptor X even if the same pi-conjugated molecule W is used.

## Claims

1. An organic optical material comprising a complex formed from
(1) a conjugated molecule having
(a) at least one electron donating site,
(b) at least one electron accepting site, and
(c) at least one conjugated site in the same molecule and
(2) a compound having a proton donating property or an electron pair accepting property, the complex having a non-covalent interaction at the electron accepting site, wherein
- the conjugated molecule does not have both one hydrogen atom and one disubstituted amino group at the same time on one of carbon atoms constituting an aliphatic carbon-carbon double bond in its molecule;
- the complex is solid at ordinary temperature; and
- the organic optical material exhibits a property of emitting light having a maximum fluorescence wavelength which causes a Stokes shift of 5% or more of the value of a maximum absorption wavelength, or of 15 nm or more or 1.5 × 10³ cm⁻¹ or more from the maximum absorption wavelength toward the long wavelength side when the conjugated molecule and the compound having a proton donating property or an electron pair accepting property form the complex, wherein
the complex is a complex represented by the following formula (1a):
W:X... (1a)
wherein W represents a conjugated molecule consisting of (A)ₗ, (B)ₘ and (C)ₙ; each A is the same or different and represents an electron donating site, selected from a hydroxy group, an alkoxy group, an alkenyl group, a silyloxy group, a silyl group, an amino group, a monosubstituted amino group, a disubstituted amino group, an alkoxycarbonyl group, a monovalent or divalent aromatic hydrocarbon group, a monovalent or divalent fused polycyclic aromatic hydrocarbon group, a monovalent or divalent saturated or unsaturated heterocyclic group, or a monovalent or divalent saturated or unsaturated fused polycyclic heterocyclic group, wherein at least one hydrogen atom of each of these groups is optionally replaced with a substituent, and when a plurality of the same or different groups are present as to each of these groups, the groups are optionally linked through a divalent atom or a divalent substituent ;
each B is the same or different and represents a conjugated site, selected from an alkenylene group, an alkynylene group, or a substituent in which at least one of these substituents is bonded to a phenylene group;
each C is the same or different and represents an electron accepting site, selected from
(1) a halogen atom,
(2) a nitro group, a phenylamino group, carbene, N-heterocyclic carbene, silylene, N-heterocyclic silylene, nitrene, N-heterocyclic nitrene, a BF₂ group, a cyano group, a pyridyl group, a pyridine N-oxy group, an acridine ring group, an acridine N-oxy group, a diazo group, a triazole group, a benzotriazole group, an oxazole group, a benzoxazole group, a thiazole group, a benzothiazole group, a diazole group, a benzodiazole group, an amino group, a monosubstituted amino group, a disubstituted amino group, a pyrazine ring group, a phenazine ring group, a pyrrolidone ring group, a pyridone ring group, an aromatic heterocyclic group, or a fused polycyclic aromatic heterocyclic group, or
(3) a formyl group, a carbonyl group, a carbodiimide group, a pyrrolidone ring group, a pyridone ring group, a formyl group, a quinone ring group, a quinoline ring group, a naphthoquinone ring group, an anthraquinone ring group, a lactone ring group, or an oxycarbonyl group;
: represents a non-covalent interaction;
X represents a compound having a proton donating property or an electron pair accepting property, which is selected from p-toluenesulfonic acid,
trifluoromethanesulfonic acid, methanesulfonic acid, N,N-dialkylimidazolinium-2-halide, halogenoisoindolyl-1,3-dione, 2-halogenobenz[d]isothiazol-3(2H)-one 1,1-dioxide, 2-halogeno-5-nitroisoindolyl-1,3-dione, or 2-halogeno-3,4-dimethylthiazol-3-inium trifluorosulfonate;
I and n each represent a number of 1 to 6;
m represents a number of 1 to 6; and
C and X are non-covalently bonded.

2. The organic optical material according to claim 1, wherein the complex is a complex represented by the following formula (1b):
W:X ... (1b)
wherein W represents a conjugated molecule consisting of (A)ₗ₁-(B)ₘ₁-(C)ₙ₁, (C)ₙ₂-(B)ₘ₂-(A)ₗ₂-(B)ₘ₃-(A)ₗ₁-(B)ₘ₁-(C)ₙ₁ or (A)ₙ₂-(B)ₘ₂-(C)ₗ₂-(B)ₘ₃-(C)ₗ₁-(B)ₘ₁-(A)ₙ₁; A represents an electron donating site as defined by claim 1; B represents a conjugated site as defined by claim 1; C represents an electron accepting site as defined by claim 1; : represents a non-covalent interaction as defined by claim 1; X represents a compound having a proton donating property or an electron pair accepting property as defined by claim 1; l1 and n1 each represent 1; m1 represents a number of 0 to 2; l2 and n2 each represent 1; m2 represents a number of 0 to 2; m3 represents 1; and C and X are non-covalently bonded.

3. Use of an organic optical material according to any one of claims 1 or 2 as an optical material which is contained in an optical composition selected from the composition group consisting of a fluorescence emitting composition, a fluorescent coating composition, a wavelength conversion member composition, an organic EL member composition, an organic laser member composition, an organic transistor member composition, an organic solar cell member composition, and a chemical sensor member composition.

4. A mixture comprising two or more organic optical materials according to any one of claims 1 or 2 mixed with each other, or an organic optical material according to any one of claims 1 or 2 mixed with an additional organic optical material.

5. A method for adjusting the color of emitted light, comprising using one or two or more organic optical materials according to any one of claims 1 or 2, or a mixture of the one or two or more organic optical materials with an additional organic optical material.

6. A fluorescent agent comprising a complex according to claim 1.

## Patentansprüche

1. Organisches optisches Material, umfassend einen Komplex, gebildet aus
(1) einem konjugierten Molekül mit
(a) wenigstens einer Elektronendonator-Stelle,
(b) wenigstens einer Elektronenakzeptor-Stelle und
(c) wenigstens einer konjugierten Stelle in demselben Molekül und
(2) einer Verbindung mit Protonendonator-Eigenschaft oder Elektronenpaarakzeptor-Eigenschaft, wobei der Komplex eine nicht-kovalente Wechselwirkung an der Elektronenakzeptor-Stelle aufweist, wobei
- das konjugierte Molekül nicht gleichzeitig sowohl ein Wasserstoffatom als auch eine disubstituierte Aminogruppe an einem der Kohlenstoffatome aufweist, die eine aliphatische Kohlenstoff-Kohlenstoff-Doppelbindung in ihrem Molekül bilden;
- der Komplex bei gewöhnlicher Temperatur fest ist;
- und wobei das organische optische Material die Eigenschaft aufweist, Licht mit einer maximalen Fluoreszenzwellenlänge zu emittieren, was eine Stokes-Verschiebung von 5 % oder mehr des Werts der maximalen Absorptionswellenlänge verursacht oder von 15 nm oder mehr oder 1,5 × 10³ cm⁻¹ oder mehr von der maximalen Absorptionswellenlänge in Richtung der langwelligen Seite, wenn das konjugierte Molekül und die Verbindung mit Protonendonator-Eigenschaft oder Elektronenpaarakzeptor-Eigenschaft den Komplex bilden, wobei der Komplex ein Komplex der folgenden Formel (1a) ist:
W:X... (1a)
wobei W für ein konjugiertes Molekül steht, das aus (A)ₗ, (B)ₘ und (C)ₙ besteht; jedes A gleich oder verschieden ist und für eine Elektronendonator-Stelle steht, ausgewählt aus einer Hydroxygruppe, einer Alkoxygruppe, einer Alkenylgruppe, einer Silyloxygruppe, einer Silylgruppe, einer Aminogruppe, einer monosubstituierten Aminogruppe, einer disubstituierten Aminogruppe, einer Alkoxycarbonylgruppe, einer einwertigen oder zweiwertigen aromatischen Kohlenwasserstoffgruppe, einer einwertigen oder zweiwertigen kondensierten polycyclischen aromatischen Kohlenwasserstoffgruppe, einer einwertigen oder zweiwertigen gesättigten oder ungesättigten heterocyclischen Gruppe oder einer einwertigen oder zweiwertigen gesättigten oder ungesättigten kondensierten polycyclischen heterocyclischen Gruppe, wobei wenigstens ein Wasserstoffatom jeder dieser Gruppen wahlweise durch einen Substituent ersetzt ist, und wenn eine Vielzahl gleicher oder verschiedener Gruppen in Bezug auf jede dieser Gruppen vorhanden ist, sind diese Gruppen wahlweise durch ein zweiwertiges Atom oder einen zweiwertigen Substituent verbunden,
jedes B gleich oder verschieden ist und für eine konjugierte Stelle steht, ausgewählt aus einer Alkenylengruppe, einer Alkinylengruppe oder einem Substituent, bei dem wenigstens einer dieser Substituenten an eine Phenylengruppe gebunden ist;
jedes C ist gleich oder verschieden ist und für eine Elektronenakzeptor-Stelle steht, ausgewählt aus
(1) einem Halogenatom,
(2) einer Nitrogruppe, einer Phenylaminogruppe, Carben, N-heterocyclischem Carben, Silylen, N-heterocyclischem Silylen, Nitren, N-heterocyclischem Nitren, einer BF₂-Gruppe, einer Cyanogruppe, einer Pyridylgruppe, einer Pyridin-N-oxygruppe, einer Acridin-Ringgruppe, einer Acridin-N-oxygruppe, einer Diazogruppe, einer Triazolgruppe, einer Benzotriazolgruppe, einer Oxazolgruppe, einer Benzoxazolgruppe, einer Thiazolgruppe, einer Benzothiazolgruppe, einer Diazolgruppe, einer Benzodiazolgruppe, einer Aminogruppe, einer monosubstituierten Aminogruppe, einer disubstituierten Aminogruppe, einer Pyrazin-Ringgruppe, Phenazin-Ringgruppe, Pyrrolidon-Ringgruppe, einer Pyridon-Ringgruppe, einer aromatischen heterocyclischen Gruppe oder einer kondensierten polycyclischen aromatischen heterocyclischen Gruppe, oder
(3) einer Formylgruppe, einer Carbonylgruppe, einer Carbodiimidgruppe, einer Pyrrolidon-Ringgruppe, einer Pyridon-Ringgruppe, einer Formylgruppe, einer Chinon-Ringgruppe, einer Chinolin-Ringgruppe, einer Naphthochinon-Ringgruppe, einer Anthrachinon-Ringgruppe, einer Lacton-Ringgruppe oder einer Oxycarbonylgruppe;
: für eine nicht-kovalente Wechselwirkung steht;
X für eine Verbindung mit Protonendonator-Eigenschaft oder Elektronenpaarakzeptor-Eigenschaft steht, die ausgewählt ist aus p-Toluolsulfonsäure, Trifluormethansulfonsäure, Methansulfonsäure, N,N-Dialkylimidazolinium-2-halogenid, 2-Halogenisoindolyl-1,3-dion, 2-Halogenbenz[d]isothiazol-3(2H)-on, 1,1-Dioxid, 2-Halogen-5-nitroisoindolyl-1,3-dion oder 2-Halogen-3,4-dimethylthiazol-3-iniumtrifluorsulfonat;
l und n jeweils für eine Zahl von 1 bis 6 stehen, m für eine Zahl von 1 bis 6 steht und C und X nicht-kovalent gebunden sind.

2. Organisches optisches Material nach Anspruch 1, wobei der Komplex ein Komplex der folgenden Formel (1b) ist:
W:X ... (1b)
wobei W für ein konjugiertes Molekül steht, bestehend aus (A)ₗ₁-(B)ₘ₁-(C)ₙ₁, (C)ₙ₂-(B)ₘ₂-(A)ₗ₂-(B)ₘ₃-(A)ₗ₁-(B)ₘ₁-(C)ₙ₁ oder (A)ₙ₂-(B)ₘ₂-(C)ₗ₂-(B)ₘ₃-(C)ₗ₁-(B)ₘ₁-(A)ₙ₁; A für eine Elektronendonator-Stelle steht, wie in Anspruch 1 definiert; B für eine konjugierte Stelle steht, wie in Anspruch 1 definiert; C für eine eine Elektronenakzeptor-Stelle steht, wie in Anspruch 1 definiert; : für eine nicht-kovalente Wechselwirkung steht, wie in Anspruch 1 definiert; X für eine Verbindung steht, die Protonendonator-Eigenschaft oder Elektronenpaarakzeptor-Eigenschaft aufweist, wie in Anspruch 1 definiert; l1 und n1 jeweils für 1 stehen; m1 für eine Zahl von 0 bis 2 steht; l2 und n2 jeweils für 1 stehen; m2 für eine Zahl von 0 bis 2 steht; m3 für 1 steht und C und X nicht-kovalent gebunden sind.

3. Verwendung eines optischen Materials nach einem der Ansprüche 1 oder 2 als optisches Material, das in einer optischen Zusammensetzung enthalten ist, ausgewählt aus der Gruppe der Zusammensetzungen, bestehend aus einer Fluoreszenzemittierenden Zusammensetzung, einer fluoreszierenden Beschichtungszusammensetzung, einer Zusammensetzung eines Wellenlängen-Umwandlungselements, einer Zusammensetzung eines organischen EL-Elements, einer Zusammensetzung eines organischen Laserelements, einer Zusammensetzung eines organischen Transistorelements, einer Zusammensetzung eines organischen Solarzellenelements und einer Zusammensetzung eines chemischen Sensorelements.

4. Mischung, bestehend aus zwei oder mehr organischen optischen Materialien nach einem der Ansprüche 1 oder 2, die miteinander gemischt sind, oder organisches optisches Material nach einem der Ansprüche 1 oder 2, das mit einem zusätzlichen organischen optischen Material gemischt ist.

5. Verfahren zum Anpassen der Farbe von emittiertem Licht, umfassend die Verwendung von einem oder zwei oder mehreren der organischen optischen Materialien nach einem der Ansprüche 1 oder 2 oder von einer Mischung von einem oder zwei oder mehreren der organischen optischen Materialien mit einem zusätzlichen organischen optischen Material.

6. Fluoreszierendes Mittel, umfassend den Komplex nach Anspruch 1.

## Revendications

1. Matériau optique organique comprenant un complexe formé à partir de
(1) une molécule conjuguée ayant
(a) au moins un site donneur d'électron,
(b) au moins un site accepteur d'électron,
(c) au moins un site conjugué dans la molécule, et
(2) un composé ayant une propriété de donneur de proton ou une propriété d'accepteur de paire d'électrons, le complexe ayant une interaction non covalente au niveau du site accepteur d'électrons, dans lequel
- la molécule conjuguée n'a pas à la fois un atome d'hydrogène et un groupe amino disubstitué en même temps sur l'un des atomes de carbone constituant une double liaison carbone-carbone aliphatique dans sa molécule ;
- le complexe est solide à la température ordinaire ; et
- le matériau optique organique présente une propriété d'émission de lumière ayant une longueur d'onde de fluorescence maximale qui provoque un décalage de Stokes de 5 % ou plus de la valeur de la longueur d'onde d'absorption maximale, ou de 15 nm ou plus ou de 1,5 × 10³ cm⁻¹ ou plus depuis la longueur d'onde d'absorption maximale vers le côté des longueurs d'onde longues quand la molécule conjuguée et le composé ayant une propriété de donneur de proton ou une propriété d'accepteur de paire d'électrons forment le complexe, dans lequel
le complexe est un complexe représenté par la formule (1a) suivante :
W:X ... (1a)
dans lequel W représente une molécule conjuguée consistant en (A)ₗ, (B)ₘ et (C)ₙ ;
chaque A est identique ou différente et représente un site donneur d'électrons choisi parmi un groupe hydroxy, un groupe alcoxy, un groupe alcényle, un groupe siloxy, un groupe silyle, un groupe amino, un groupe amino monosubstitué, un groupe amino disubstitué, un groupe alcoxycarbonyle, un groupe hydrocarboné aromatique monovalent ou divalent, un groupe hydrocarboné aromatique polycyclique condensé monovalent ou divalent, un groupe hétérocyclique saturé ou insaturé monovalent ou divalent, ou un groupe hétérocyclique polycyclique condensé saturé ou insaturé monovalent ou divalent, dans lequel au moins un atome d'hydrogène de chacun de ces groupes est optionnellement remplacé par un substituant, et quand plusieurs des groupes identiques ou différents sont présents en tant que chacun de ces groupes, les groupes sont optionnellement liés par l'intermédiaire d'un atome divalent ou d'un substituant divalent ;
chaque B est identique ou différent et représente un site conjugué choisi parmi un groupe alcénylène, un groupe alcynylène, ou un substituant dans lequel au moins l'un de ces substituants est lié à un groupe phénylène ;
chaque C est identique ou différent et représente un site accepteur d'électron choisi parmi
(1) un atome d'halogène;
(2) un groupe nitro, un groupe phénylamino, un carbène, un carbène N-hétérocyclique, un silylène, un silylène N-hétérocyclique, un nitrène, un nitrène N-hétérocyclique, un groupe BF₂, un groupe cyano, un groupe pyridyle, un groupe pyridine-N-oxy, un groupe cyclique acridine, un groupe acridine-N-oxy, un groupe diazo, un groupe triazole, un groupe benzotriazole, un groupe oxazole, un groupe benzoxazole, un groupe thiazole, un groupe benzothiazole, un groupe diazole, un groupe benzodiazole, un groupe amino, un groupe amino monosubstitué, un groupe amino disubstitué, un groupe cyclique pyrazine, un groupe cyclique phénazine, un groupe cyclique pyrrolidone, un groupe cyclique pyridone, un groupe hétérocyclique aromatique, ou un groupe hétérocyclique aromatique polycyclique condensé, et
(3) un groupe formyle, un groupe carbonyle, un groupe carbodiimide, un groupe cyclique pyrrolidone, un groupe cyclique pyridone, un groupe formyle, un groupe cyclique quinone, un groupe cyclique quinoline, un groupe cyclique naphtoquinone, un groupe cyclique anthraquinone, un groupe cyclique lactone, ou un groupe oxycarbonyle ;
: représente une interaction non covalente ;
X représente un composé ayant une propriété de donneur de proton ou une propriété d'accepteur de paire d'électrons, qui est choisi parmi l'acide p-toluènesulfonique, l'acide trifluorométhanesulfonique, l'acide méthanesulfonique, un 2-halogénure de N,N-dialkylimidazolinium, une halogénoisoindolyl-1,3-dione, un 1,1-dioxyde de 2-halogénobenz[d]isothiazol-3(2H)-one, une 2-halogéno-5-nitroisoinsolyl-1,3-dione, et un trifluorosulfonate de 2-halogéno-3,4-diméthylthiazol-3-inium ;
l et n représentent chacun un nombre de 1 à 6 ;
m représente un nombre de 1 à 6 ; et
C et X sont liés de manière non covalente.

2. Matériau optique organique selon la revendication 1, dans lequel le complexe est un complexe représenté par la formule (1b) suivante :
W:X ... (1b)
dans lequel W représente une molécule conjuguée consistant en (A)ₗ₁-(B)ₘ₁-(C)ₙ₁, (C)ₙ₂-(B)ₘ₂-(A)ₗ₂-(B)ₘ₃-(A)ₗ₁-(B)ₘ₁-(C)ₙ₁ ou (A)ₙ₂-(B)ₘ₂-(C)ₗ₂-(B)ₘ₃-(C)ₗ₁-(B)ₘₗ-(A)ₙ₁ ; A représente un site donneur d'électrons tel que défini dans la revendication 1 ; B représente un site conjugué tel que défini dans la revendication 1 ; C représente un site accepteur d'électron tel que défini dans la revendication 1 ; : représente une interaction non covalente telle que définie dans la revendication 1 ; X représente un composé ayant une propriété de donneur de proton ou une propriété d'accepteur de paire d'électrons tel que défini dans la revendication 1 ; l1 et n1 représentent chacun 1 ; m1 représente un nombre de 0 à 2 ; l2 et n2 représentent chacun 1 ; m2 représente un nombre de 0 à 2 ; m3 représente 1 ; et C et X sont liés de manière non covalente.

3. Utilisation d'un matériau optique organique selon l'une quelconque des revendications 1 et 2 en tant que matériau optique qui est contenu dans une composition optique choisie dans le groupe de compositions consistant en une composition émettant une fluorescence, une composition d'enrobage fluorescente, une composition d'élément de conversion de longueur d'onde, une composition d'élément EL organique, une composition d'élément de laser organique, une composition d'élément de transistor organique, une composition d'élément de cellule solaire organique, et une composition d'élément de capteur chimique.

4. Mélange comprenant deux ou plus de deux matériaux optiques organiques selon l'une quelconque des revendications 1 et 2 mélangés ensemble, ou un matériau optique organique selon l'une quelconque des revendications 1 et 2 mélangé à un matériau optique organique additionnel.

5. Procédé pour ajuster la couleur d'une lumière émise, comprenant l'utilisation d'un ou deux ou plus de deux matériaux optiques organiques selon l'une quelconque des revendications 1 et 2, ou d'un mélange du ou des deux ou des plus de deux matériaux optiques organiques avec un matériau optique organique additionnel.

6. Agent fluorescent comprend un complexe selon la revendication 1.
